# EUROPEAN PATENT APPLICATION

(11) **EP 4 497 471 A2**
(43) Date of publication of application: **29.01.2025**
(21) Application number: 24218932.2
(22) Date of filing: 19.10.2020
(51) Int. Cl.: A61N 7/00

(54) **STIMULATION OF NEURONAL PLASTICITY**

(30) Priority: 18.10.2019 EP 19204173; 04.09.2020 EP 20194579
(62) Divisional of application: 20792659.3
(71) Applicant: Institute of Science and Technology Austria, 3400 Klosterneuburg (AT)
(72) Inventor: VENTURINO, Alessandro, Klosterneuburg (AT); SIEGERT, Sandra, Klosterneuburg (AT)
(74) Representative: Script Intellectual Property LLP

(57) **Abstract**

A stimulus-emitting device configured to promote neuronal plasticity by induction of in vivo synchronized gamma oscillations and removal of the perineuronal net in at least one brain region of a subject; wherein the device comprises a light source configured to emit flashing light at a frequency of about 50 to about 70 Hz; and wherein the device is configured to induce synchronized gamma oscillations of a frequency of about 50 to about 70 Hz in the brain region of the subject by means of the flashing light.

## Description

### FIELD OF THE INVENTION

The present invention relates to the promotion of plasticity in the central nervous system, especially in the brain. In particular, the invention relates to promoting neuronal plasticity by removal of the perineuronal net. The invention may be particularly useful for the treatment of conditions and diseases where it is desirable to promote neuronal plasticity and/or to remove or reduce the perineuronal net. Such conditions include those where neuronal connections are compromised, e.g. depression and schizophrenia and spinal cord injuries.

### BACKGROUND TO THE INVENTION

Ketamine is a potent anaesthetic and analgesic and acts prominently on GABAergic inhibitory neurons. It is a dissociative drug with a wide range of application in anaesthesia, analgesia, sedation, as well as treating psychiatric symptoms (Krystal, J. H. et al (2019) Neuron 101(5):774-778; Li, L. & Vlisides, P. E. (2016) Front. Hum. Neurosci. 10:612; Zanos, P. et al (2018) Pharmacol. Rev. 70(3):621-660). Repeated long-term ketamine treatment is beneficial for patients with neuropathic pain or depression (Krystal, J. H. *et al (supra);* Goldberg, M. E. et al (2010) Pain Physician 13(4):379-87; Kiefer, R. T. et al (2008) Pain Med. 9(1):44-54; Becerra, L. et al (2015) Pain Med. (United States) 16(12):2368-85; Berman, R. M. et al (2000) Biol. Psychiatry 47(4):351-4) but, on the other hand, leads to anxiety and memory impairment in healthy individuals (Morgan, C. J. A. et al (2014) Front. Psychiatry 5:149; Adler, C. M. et al(1998) Biol. Psychiatry 43(11):811-6; Hohlbaum, K. et al (2018) PLoS One 13(9):e0203559; Strong, C. E. & Kabbaj, M. (2018) Neurobiology of Stress 9:166-175). Repeated ketamine exposure is currently used to treat depression.

It is believed that these effects are caused by ketamine's antagonistic action on NMDA receptors. However, the observed neuronal effects of increased spine numbers and excitation in pyramidal neurons are inconsistent with this notion (Krystal, J. H. *et al (supra);* Li, L. & Vlisides, P. E. (*supra*); Zanos, P. *et al* (*supra*); Seamans, J. (2008) Nature Chemical Biology. 4(2):91-3; Behrens, M. M. et al. (2007) Science 318(5856):1645-7; Citri, A. & Malenka, R. C. (2008) Neuropsychopharmacol. 33(1):18-41; Paoletti, P. et al (2013) Nature Rev. Neurosci. 14(6):383-400; Derkach, V. A. et al (2007) Nature Reviews Neurosci. 8(2):101-13).

Ketamine preferentially acts on NMDA receptors of GABAergic inhibitory interneurons (Krystal, J. H. *et al (supra);* Li, L. & Vlisides, P. E. (*supra*); Zanos, P. *et al* (*supra*); Brown, E. N. et al (2011) Annu. Rev. Neurosci. 34:601-28; Picard, N. et al (2019) Mol. Psychiatry 24(6):828-838). A subpopulation of those in the cortex are the fast-spiking parvalbumin neurons, whose activity depends on the perineuronal net (PNN), an extracellular matrix compartment that constrains synaptic plasticity (Tewari, B. P. et al (2018) Nat. Commun. 9(1):4724; Celio, M. R. & Blumcke, I. (1994) Brain Res. Rev. 19(1):128-145).

Perineuronal nets (PNNs) are specialised extracellular matrix structures responsible for multiple functions, including regulating synaptic plasticity, protecting neurons from oxidative stress and neurotoxins and synaptic stabilisation in the adult brain (Flores, C.E.; Méndez, P. (2014). Frontiers in Cellular Neuroscience 8:327). PNNs are found around certain neuron cell bodies and proximal neurites in the central nervous system. Through their physiological roles, PNNs are also involved in cognition - including encoding, maintaining, and updating memories. Permanent removal of PNNs can render neurons vulnerable to damage - particularly in neurodegenerative conditions involving increased oxidative stress or neurotoxins. Current strategies have applied complete removal of the extracellular matrix in animal models using chondronitase ABC, which also digests the PNN, and has shown to increase plasticity, leading to enhanced memory interference from competing information during the encoding process. Environmental factors, such as physical activity, drugs, and nutrition, can influence brain plasticity, and some of these effects may be mediated by changes in PNN structure.

PNNs play a critical role in the closure of the childhood critical period and their digestion can cause restored critical period-like synaptic plasticity in the adult brain. Due to the biochemical composition, the PNN is largely negatively charged and composed of chondroitin sulphate proteoglycans, molecules that play a key role in development and plasticity during postnatal development and in the adult.

PNNs appear to be mainly present in the cortex, hippocampus, thalamus, brainstem, and the spinal cord. Studies of the rat brain have shown that the cortex contains high numbers of PNNs in the motor and primary sensory areas and relatively fewer in the association and limbic cortices (Galtrey, C. M. & Fawcett, J. W. (2007) Brain Research Reviews 54(1): 1-18). In the cortex, PNNs are associated mostly with inhibitory interneurons and are thought to be responsible for maintaining the excitatory/inhibitory balance in the adult brain (Celio, M. R. et al (1998). Trends in Neurosciences 21(12): 510-515).

Reductions in neuronal plasticity have been implicated in various neurological disorders. For instance, stress-induced changes in neural plasticity have been linked to depression (Duman et al. Nat Med. 2016 Mar; 22(3): 238-249). A decrease in brain plasticity could underlie age-related changes including cognitive decline (Mahncke et al., Progress in Brain Research, Volume 157, 2006, Pages 81-109). In schizophrenia, there is evidence for disrupted neuroplasticity resulting in cortical inhibition and dysfunctional intracortical connectivity (Bhandari et al., Front Psychiatry. 2016; 7: 45).

WO 2017/091698 and WO 2019/075094 disclose systems and methods for treating dementia or Alzheimer's disease by inducing synchronized gamma oscillations in the brain of a subject. The gamma oscillations may be induced by e.g. a visual stimulus at a particular frequency. However the methods in these publications typically induce low gamma frequency oscillations (e.g. about 40 Hz). WO 2017/091698 and WO 2019/075094 do not describe methods or systems specifically adapted for promoting neuronal plasticity or for treating conditions such as depression and schizophrenia, e.g. by removal of the PNN.

There is therefore a need for new methods for promoting neuronal plasticity, e.g. by removal of the perineuronal net. Such methods are likely to be useful for treating neurological conditions including depression and schizophrenia.

### SUMMARY OF THE INVENTION

The present inventors have surprisingly demonstrated that neuronal plasticity can be promoted by inducing high gamma frequency neuronal oscillations in the brain, e.g. in the range of about 50 to about 70 Hz. Induction of such high gamma oscillations may result in PNN loss, mediated by microglia. In particular embodiments, ketamine or a visual stimulus (e.g. a flashing light at a frequency of about 50 to 70 Hz) may be used to induce suitable neuronal oscillations, and thus to promote PNN loss and neuronal plasticity.

Accordingly in one aspect, the present invention provides a method for promoting neuronal plasticity in a subject, comprising inducing synchronized gamma oscillations in at least one central nervous system (e.g. brain) region of the subject; wherein the synchronized gamma oscillations have a frequency of about 50 to about 70 Hz.

In one embodiment, the synchronized gamma oscillations induce or promote removal of the perineuronal net. Preferably the synchronized gamma oscillations induce removal of the perineuronal net surrounding neurons, preferably parvalbumin-positive interneurons, more preferably cortical parvalbumin-positive interneurons.

In one embodiment, the method comprises applying a visual stimulus to the subject, e.g. in order to induce the synchronized gamma oscillations. The visual stimulus may comprise a flashing light, e.g. having a frequency of about 50 to about 70 Hz, about 55 to about 65 Hz or about 57 to about 63 Hz.

In alternative embodiments, the method comprises administering a pharmaceutical composition comprising ketamine to the subject, e.g. in order to induce the synchronized gamma oscillations.

In one embodiment, the central nervous system (CNS) region comprises a brain region. In another embodiment, the CNS region comprises the spinal cord. The brain region may comprise the cortex, preferably sensory cortex, more preferably primary visual cortex or primary somatosensory cortex.

The methods may be used to prevent or treat a neuropsychiatric disorder, e.g. schizophrenia, bipolar disorder, post-traumatic stress disorder (PTSD), and/or depression.

In a further aspect, the present invention provides a method for preventing or treating a neuropsychiatric disorder (e.g. schizophrenia, bipolar disorder, PTSD and/or depression) in a subject, comprising applying a sensory stimulus to the subject at a frequency of about 50 to about 70 Hz. Preferably the sensory stimulus is a visual stimulus or an auditory stimulus, more preferably a flashing light or light pulses at a frequency of about 50 to about 70 Hz.

In another aspect, the present invention provides a method for promoting cognitive function in a subject. The method may comprise inducing synchronized gamma oscillations in at least one brain region of the subject, e.g. by applying a visual stimulus comprising a flashing light at about 50 to about 70 Hz to the subject.

The cognitive function preferably comprises learning, attention or memory. In one embodiment the subject is a normal subject. In another embodiment, the method is a non-therapeutic method. Preferably the subject is experiencing stress, e.g. chronic stress.

In a further aspect, the present invention provides a method for inducing gamma wave entrainment at about 50 to 70 Hz in the CNS (e.g. brain) of a subject, comprising (i) applying a visual stimulus to the subject at a frequency of about 50 to 70 Hz; (ii) monitoring gamma wave entrainment in the CNS (e.g. brain) of the subject; and (iii) continuing application of the visual stimulus until gamma wave entrainment has been achieved for a predetermined time period.

In another aspect, the present invention provides a stimulus-emitting device configured to promote neuronal plasticity by induction *of in vivo* synchronized gamma oscillations in at least one CNS (e.g. brain) region of a subject. Typically the device comprises a light source configured to emit flashing light at a frequency of about 50 to about 70 Hz. The device may be configured to induce synchronized gamma oscillations of a frequency of about 50 to about 70 Hz in the brain region of the subject by means of the flashing light.

In one embodiment, the light source is configured to emit flashing light at a frequency of about 55 to about 65 Hz or about 57 to about 63 Hz. The light source may comprises e.g. an array of light emitting diodes. In a further embodiment, the device may comprise a timer connected to the light source to enable the light source to emit light for a selected period of time. The device is preferably configured to emit light having an intensity of about 1 - 8 x 10¹⁸ photons/cm²/s.

In one embodiment the device further comprises a sound source, wherein the sound source is configured to promote the induction of the synchronized gamma oscillations at a frequency of about 50 to about 70 Hz in the subject's brain. The sound source may be configured to emit sound pulses at a frequency of about 50 to about 70 Hz.

The stimulus-emitting device may be used e.g. to prevent or treat a neuropsychiatric disorder, e.g. in preventing or treating schizophrenia, bipolar disorder, PTSD and/or depression. Thus the present disclosure also provides a method of treatment of such conditions using the device, and use of the stimulus-emitting device to treat such conditions in a subject in need thereof.

In a further aspect, the present invention provides a method of operating a stimulus-emitting device as defined herein. The method may comprise generating light pulses at a frequency of about 50 to about 70 Hz (e.g. about 55 to about 65 Hz), and directing the light pulses towards a subject (preferably towards the eyes of the subject). The subject may be e.g. a normal subject, or a subject suffering from a neuropsychiatric disorder.

In a further aspect, the present invention provides ketamine, or a pharmaceutical composition comprising ketamine, for use in (i) inducing synchronized gamma oscillations in at least one CNS (e.g. brain) region of a subject; wherein the synchronized gamma oscillations have a frequency of about 50 to about 70 Hz; (ii) promoting removal of the perineuronal net in a CNS (e.g. brain) region of a subject; and/or (iii) promoting neuronal plasticity in a CNS (e.g. brain) region of a subject.

In a further aspect, the present invention provides use of ketamine for the preparation of a medicament for (i) inducing synchronized gamma oscillations in at least one CNS (e.g. brain) region of a subject; wherein the synchronized gamma oscillations have a frequency of about 50 to about 70 Hz; (ii) promoting removal of the perineuronal net in a CNS (e.g. brain) region of a subject; and/or (iii) promoting neuronal plasticity in a CNS (e.g. brain) region of a subject.

In a further aspect, the present invention provides light pulses or photons at a frequency of about 50 to 70 Hz, for use in (i) inducing synchronized gamma oscillations in at least one CNS (e.g. brain) region of a subject; wherein the synchronized gamma oscillations have a frequency of about 50 to about 70 Hz; (ii) promoting removal of the perineuronal net in a CNS (e.g. brain) region of a subject; and/or (iii) promoting neuronal plasticity in a CNS (e.g. brain) region of a subj ect.

In a further aspect, the present invention provides light pulses or photons for use in (i) inducing synchronized gamma oscillations in at least one CNS (e.g. brain) region of a subject; wherein the synchronized gamma oscillations have a frequency of about 50 to about 70 Hz; (ii) promoting removal of the perineuronal net in a CNS (e.g. brain) region of a subject; and/or (iii) promoting neuronal plasticity in a CNS (e.g. brain) region of a subject; wherein the light pulses or photons are applied to the subject at a frequency of about 50 to 70 Hz.

In a further aspect, the present invention provides use of light pulses or photons at a frequency of about 50 to 70 Hz in the preparation of an agent or therapy for (i) inducing synchronized gamma oscillations in at least one CNS (e.g. brain) region of a subject; wherein the synchronized gamma oscillations have a frequency of about 50 to about 70 Hz; (ii) promoting removal of the perineuronal net in a CNS (e.g. brain) region of a subject; and/or (iii) promoting neuronal plasticity in a CNS (e.g. brain) region of a subject.

In any of the above aspects, the ketamine or light pulses may be applied to treat a neuropsychiatric disorder, e.g. schizophrenia, bipolar disorder, PTSD and/or depression, preferably in a sub-group of patients in which it is desirable to promote neuronal plasticity. For instance, the treatment may be applied to treating subjects showing dysfunctional neuronal plasticity, dysfunctional gamma frequency oscillations or a dysfunctional perineuronal net.

In another aspect, the present invention provides a pharmaceutical composition comprising an active agent for use in a method of treating or preventing a neuropsychiatric disorder in a subject. The neuropsychiatric disorder may be e.g. schizophrenia, anxiety, bipolar disorder and/or depression. Preferably the method further comprises inducing synchronized gamma oscillations in at least one brain region of the subject, e.g. by applying a visual stimulus comprising a flashing light at about 50 to about 70 Hz to the subject.

In some embodiments, the active agent comprises an antidepressant, an anxiolytic, a psychedelic, an antipsychotic or a neuroleptic drug. Preferably the visual stimulus is applied to the subject at intervals between treatments with the active agent, thereby prolonging or sustaining a response to the active agent.

In a further aspect, the present invention provides a method for treating or preventing a neuropsychiatric disorder in a subject; the method comprising inducing synchronized gamma oscillations in at least one brain region of the subject by applying a visual stimulus comprising a flashing light at about 50 to about 70 Hz to the subject. Preferably the neuropsychiatric disorder is schizophrenia, anxiety, bipolar disorder and/or depression.

In a further aspect, the present invention provides a method for sustaining or prolonging a response to an active agent used to treat or prevent a neuropsychiatric disorder in a subject; the method comprising inducing synchronized gamma oscillations in at least one brain region of the subject by applying a visual stimulus comprising a flashing light at about 50 to about 70 Hz to the subject. Preferably the neuropsychiatric disorder is schizophrenia, anxiety, bipolar disorder and/or depression.

In one embodiment, the visual stimulus is applied to the subject for less than one hour daily, preferably for 1 to 30 minutes or about 5 minutes daily. The visual stimulus is preferably applied to the subject during mental or cognitive challenges or exercises, monocular deprivation, fear extinction training, psychosocial therapy, learning and relearning, psychotherapy, behavioural therapy, trauma therapy, or exposure and response prevention (ERP) therapy. Preferably the visual stimulus is applied to the subject, e.g. daily, between treatments with the active agent.

The active agent may be administered to the subject in a clinically-supervised environment, and/or the visual stimulus may be applied to the subject in an unsupervised or home environment. Preferably the active agent comprises ketamine, esketamine or a psychedelic drug.

In a further aspect, the present invention provides a system for promoting neuronal plasticity in at least one brain region of a subject, comprising (i) a stimulus-emitting device as described above, and (ii) a monitoring device for monitoring synchronized gamma oscillations in the brain region of the subject.

In one embodiment, the system further comprises a processor configured to modulate a duration, frequency and/or intensity of the flashing light emitted by the light source in response to detection of synchronized gamma oscillations of a frequency of about 50 to about 70 Hz by the monitoring device. In another embodiment, the system further comprises a user interface for displaying brain activity detected by the monitoring device to a user. Preferably the monitoring device is an electroencephalogram (EEG) apparatus.

### BRIEF DESCRIPTION OF THE DRAWINGS

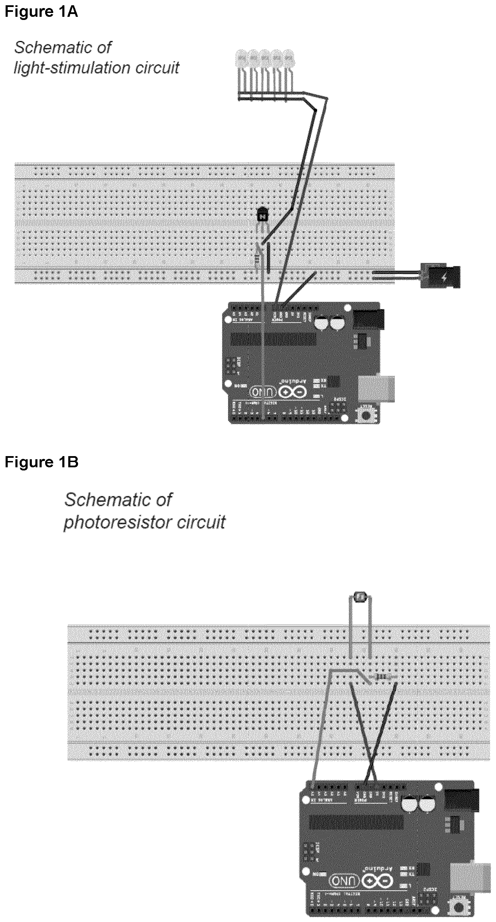

**Figure 1****-** Schematic of light-stimulation circuit (A) and photoresistor circuit (B) as described in the examples.

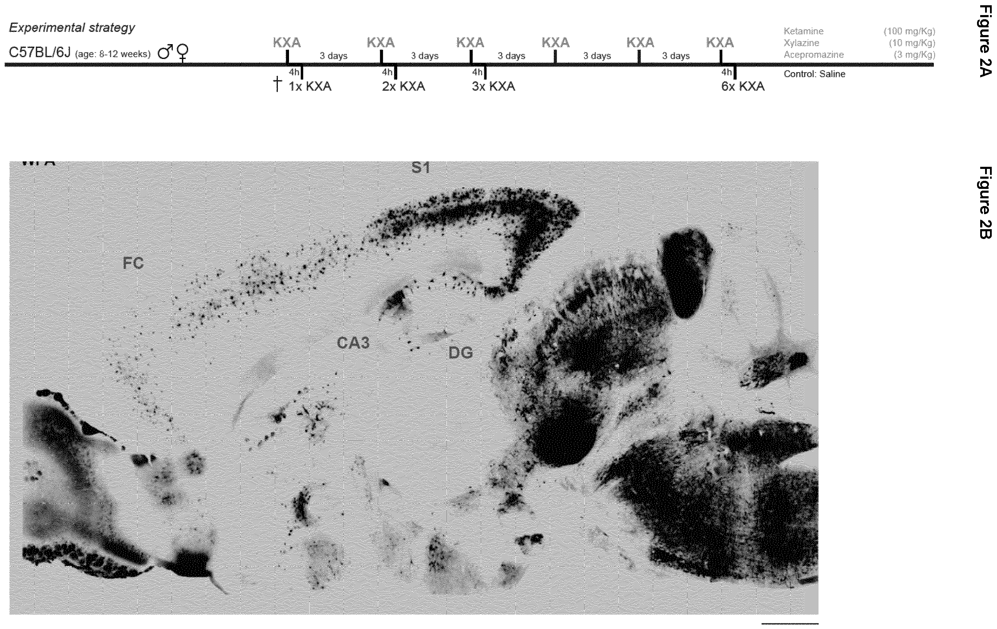

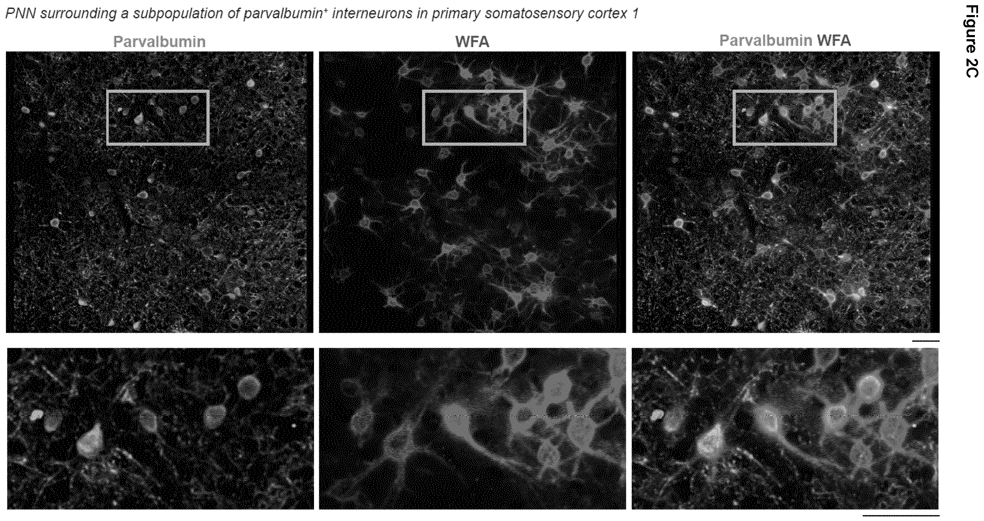

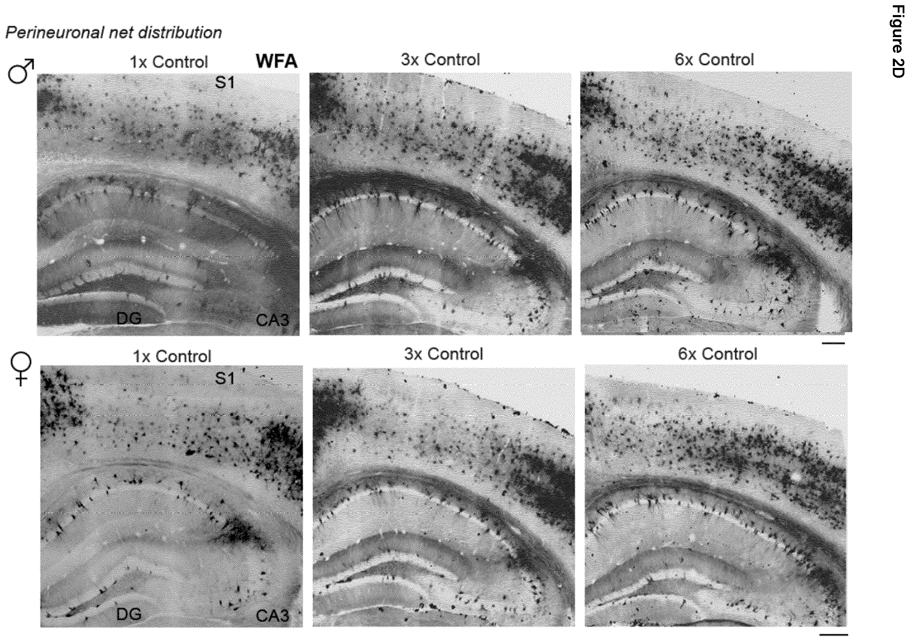

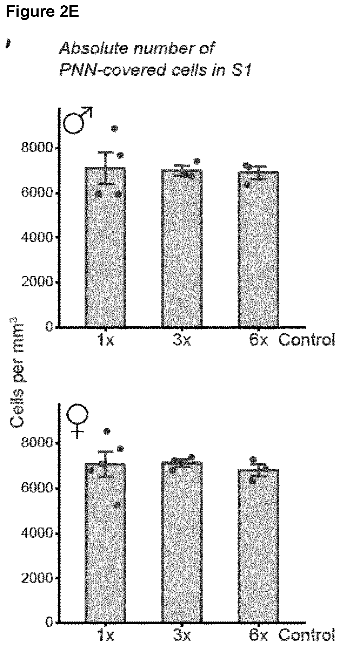

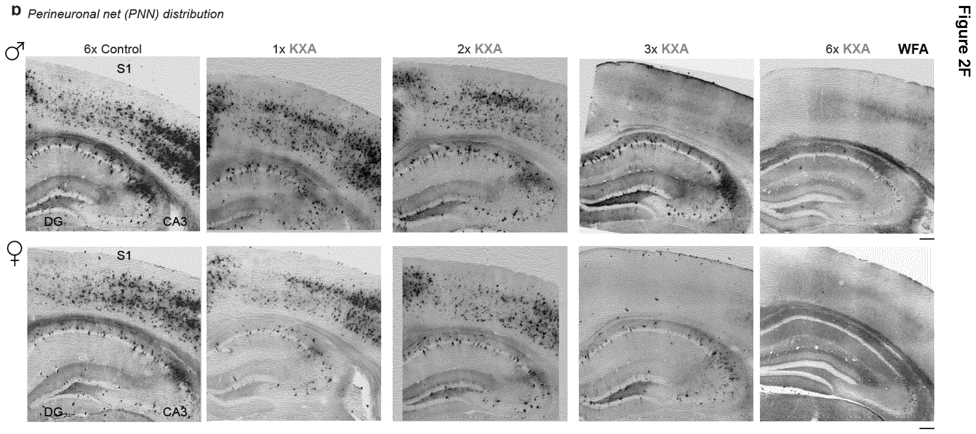

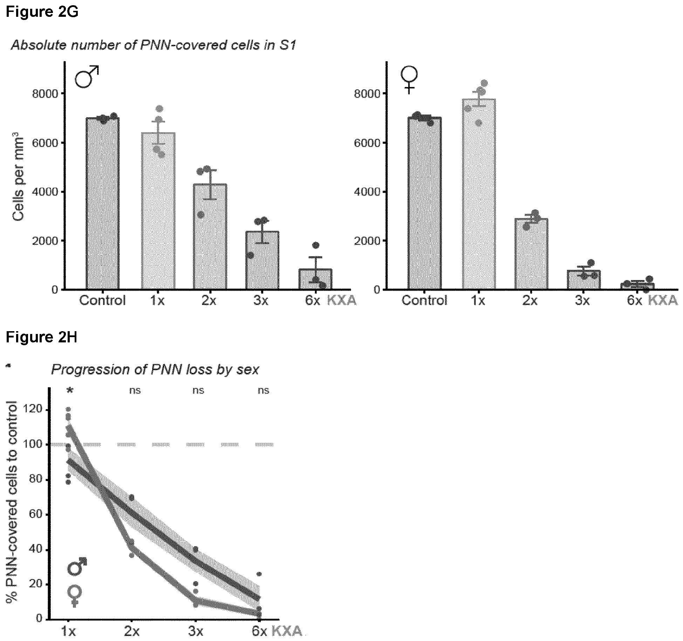

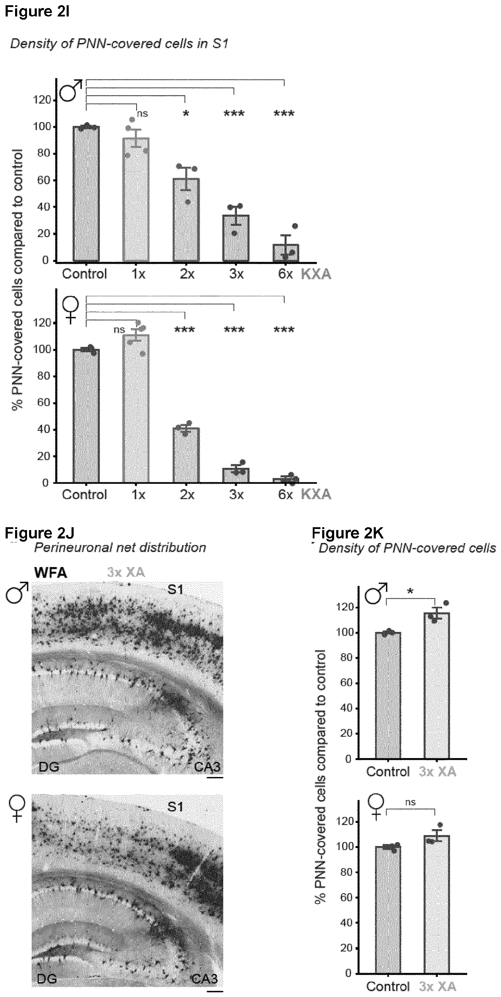

**Figure 2** - Repeated exposure to ketamine results in PNN loss. **Figure 2A****:** Schematic of experimental strategy in adult male and female C57BL6/J animals. **Figures 2B-2C****:** PNN distribution in in the cortex. Representative maximum intensity projection (MIP) overview image immunostained with WFA (*Wisteria floribunda* agglutinin), colour-inverted in parasagittal (Figure 2B) and magenta in coronal (Figure 2C) brain section. Scale bar: 1000 µm and 300 µm, respectively. **Figure 2C****:** Zoom-in MIP image into primary somatosensory cortex showing parvalbumin-positive interneurons (cyan) surrounded by WFA (arrows). Scale bar: 50 µm. CA3, *corno ammonis* region 3. DG, dentate gyrus. FC, frontal cortex. S1, primary somatosensory cortex. V1, primary visual cortex. **Figure 2D****:** Coronal brain sections immunostained for WFA (*Wisteria floribunda* agglutinin, color-inverted) for 1x, 3x and 6x saline injection in male (top) and female (bottom). CA3, *corno ammonis* region 3, DG, dentate gyrus. S1, primary somatosensory cortex. Scale bar: 200 µm. **Figure 2E****:** Bar chart of absolute number of PNN-covered cells after 1x, 3x, 6x saline injections. 3-4 animals per condition. Linear regression model: p _{♂}=0.967. p _{♀}=0.902. **Figure 2F****:** Coronal brain sections immunostained for WFA after 6x Control (saline), 1x, 2x, 3x, and 6x KXA in male (top) and female (bottom). **Figure 2G****:** Bar chart of absolute number of PNN-covered cells in the S1 compared to combined control for male (left) and female (right). **Figure 2H****:** Line chart comparing the progression of PNN loss for each sex. Linear regression model: p=1.75*10⁻¹¹ with shown post-hoc values comparing the progression of PNN loss for each sex. **Figure 2I**: Bar chart of per cent PNN-covered cells in the S 1 compared to combined control for male (top) and female (bottom). 3-5 animals per condition. Linear regression model: p_{♂} = 5.35*10⁻⁶ and p_{♀}= 2.98*10⁻¹¹, with post-hoc in graph. **Figure 2J****:** Coronal brain sections immunostained for WFA after 3x XA exposure in male (top) and female (bottom). **Figure 2K****:** Bar chart of per cent PNN-covered cells in the S1 for 3x XA compared to combined control for male (top) and female (bottom). 3-5 animals per condition. Two sample t-test: p_{♂}= 0.048. p _{♀}= 0.12. For control, see Figures 2D and 2E. CA3, *corno ammonis* region 3, DG, dentate gyrus. KXA, ketamine-xylazine-acepromazine. PNN, perineuronal net. S1, primary somatosensory cortex. WFA, *Wisteria floribunda* agglutinin. XA, xylazine-acepromazine. Scale bar: 200 µm.

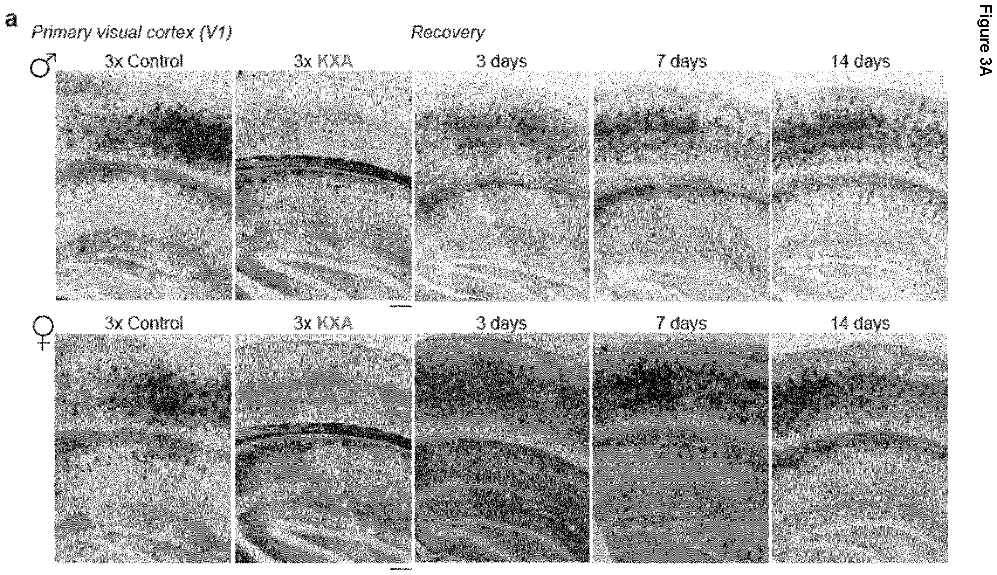

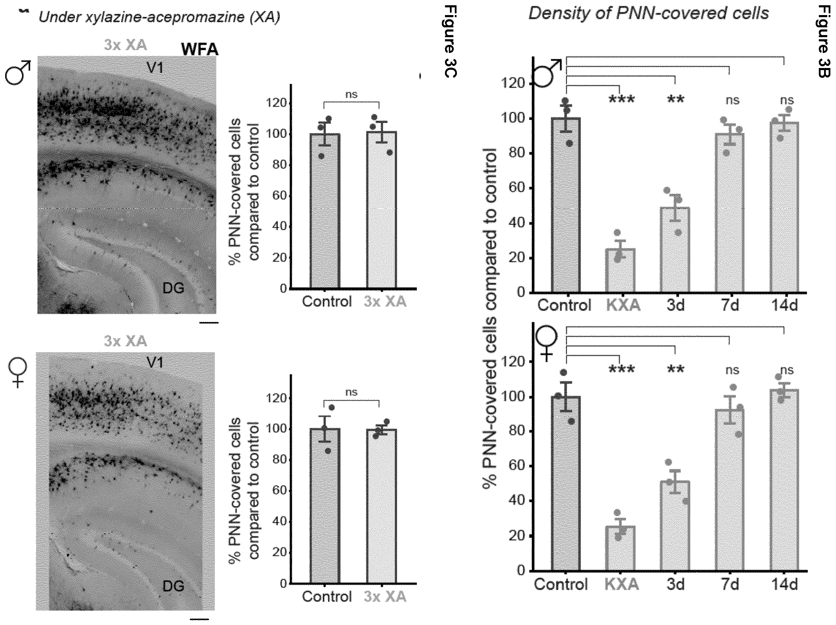

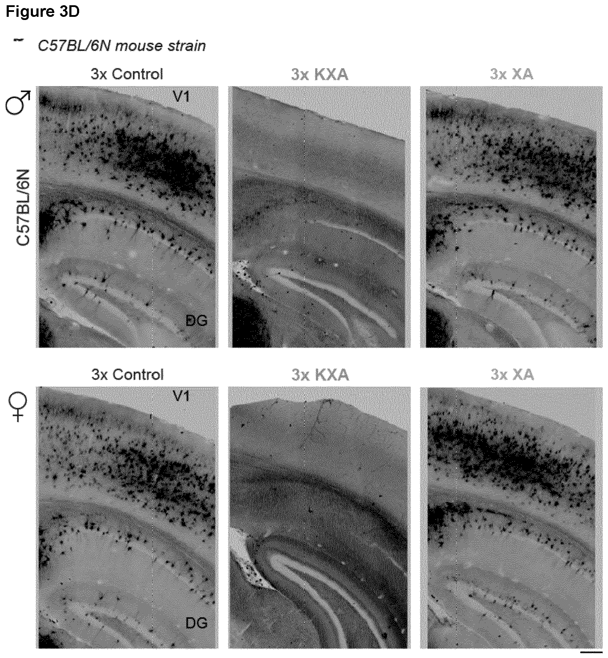

**Figure 3** - Repeated ketamine exposure temporarily reopens plasticity in the primary visual cortex (V1). **Figure 3A****:** Coronal brain sections immunostained for WFA after 3x saline (Control), 3x KXA, and 3, 7, and 14 days recovery from the last injection of 3x KXA in male (top) and female (bottom). **Figure 3B****:** Bar chart of per cent PNN-covered cells. 3 animals per condition. Linear regression model: p_{♂}= 1.39*10⁻⁵. p_{♀}= 1.67*10⁻⁵ with post-hoc shown in graph. **Figures 3C-3D****:** XA or mouse strain background does not impact PNN loss in primary visual cortex (V1). Coronal brain sections for male (upper row) and female (lower row) immunostained for WFA. **Figure 3C** after 3x XA injection in C57BL/6J, and **Figure 3D****,** 3x saline, KXA, or XA injection in C57BL/6N. Scale bar: 200 µm. Next to a, bar chart of per cent PNN-covered cells. 3 animals per condition. Two sample t-test: p_{♂}= 0.95. p _{♀}= 0.95. DG: dentate gyrus; KXA: ketamine-xylazine-acepromazine; V1: primary visual cortex; XA: xylazine-acepromazine.

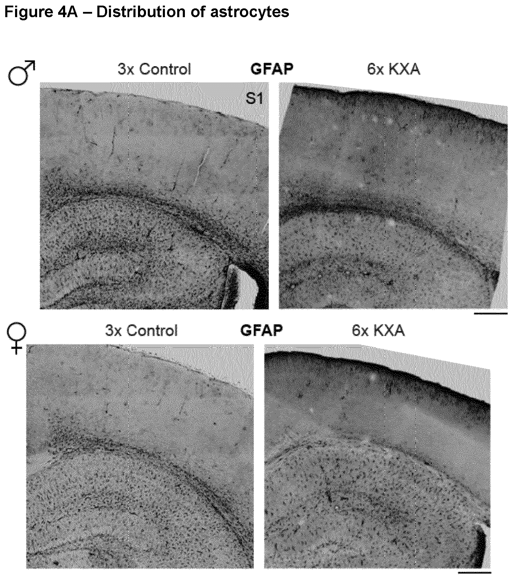

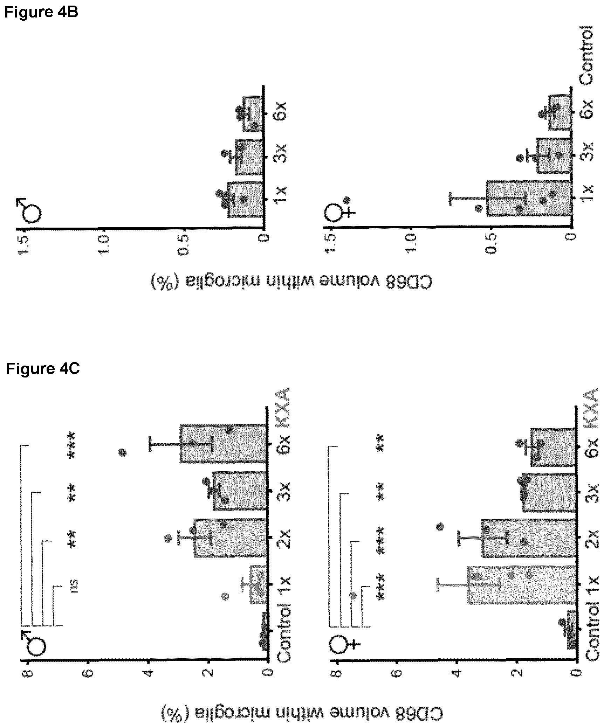

**Figure 4** - Repeated ketamine exposure does not induce apoptosis or alteration in parvalbumin-positive neuron density, nor induce astrogliosis, but turns microglia to be phagocytotic in the primary somatosensory cortex (S1). **Figure 4A****:** MIP for glial fibrillary acidic protein (GFAP) for 6x saline (control) or 6x KXA injection. Scale bar: 300 µm. **Figure 4B** Bar chart of CD68 volume within microglia in saline injections. 3-5 animals per condition. Linear regression model: p_{♂}=0.15. p _{♀}=0.34. **Figure 4C** Bar chart of CD68 volume within microglia in repeated KXA-injections. 3-5 animals per condition. Linear regression model: p_{♂}=2.78*10⁻⁴. p _{♀}=1.52*10⁻⁴ with selected post-hoc comparison.

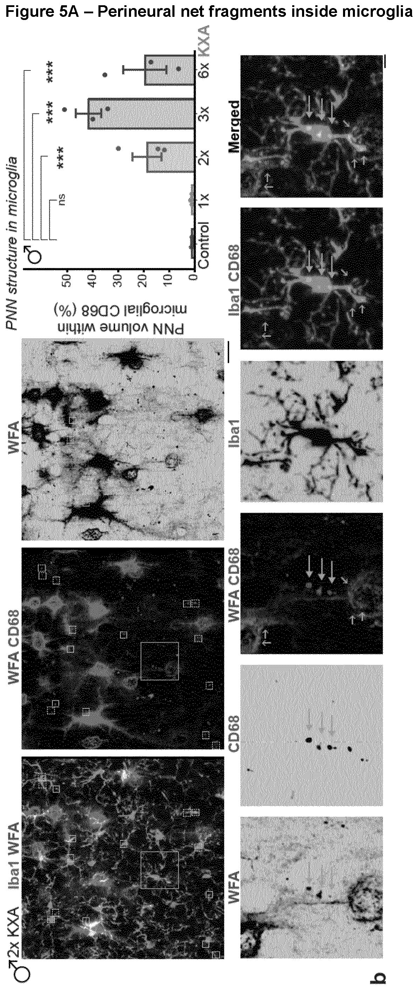

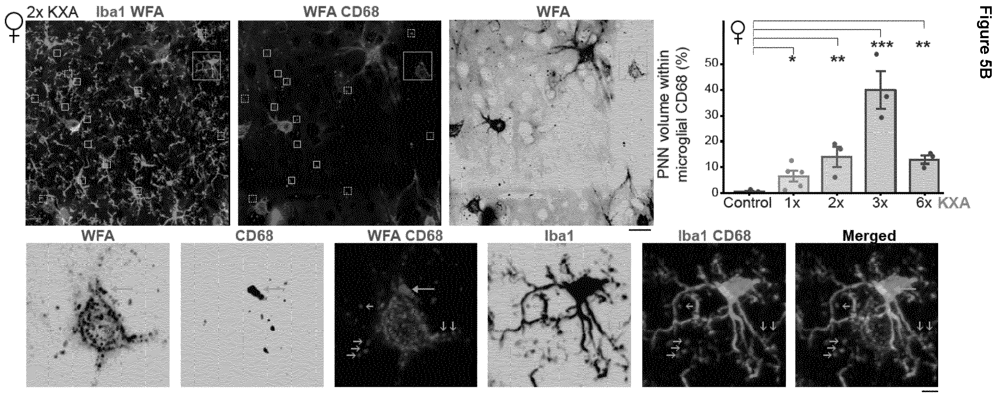

**Figure 5** **-** Microglia remove PNN upon repeated KXA exposure in primary somatosensory cortex (S1). **Figures 5A-5B****:** Representative maximum intensity projection (MIP) overview image showing immunostained PNN (WFA, magenta), microglia (Iba1, green), and endosomal-lysosomal marker CD68 (blue) in S1 after 2x KXA for male (**Figure 5A**) and female (**Figure 5B**). Dashed frames: CD68/WFA colocalisations within field of view. Scale bar: 20 µm. Full frame: zoom-in below. Closed arrow head, CD68/WFA colocalizations. Open arrow head, microglia process wrapping PNN structure. Scale bar: 5 µm. Next, bar chart showing percentage of PNN volume within microglial CD68. Each dot represents an animal. 3-5 animals for each condition. Linear regression model: **Figure 5A****:** p_{♂}= 1.46*10⁻⁶. **Figure 5B****:** p_{♀}= 1.6*10⁻⁴ with selected post-hoc in graph.

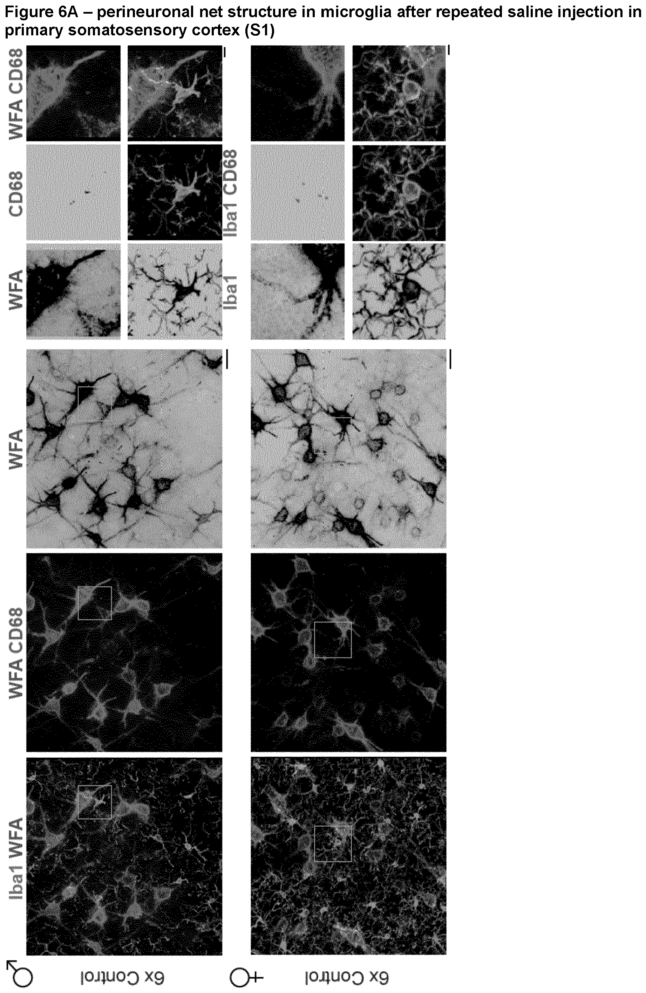

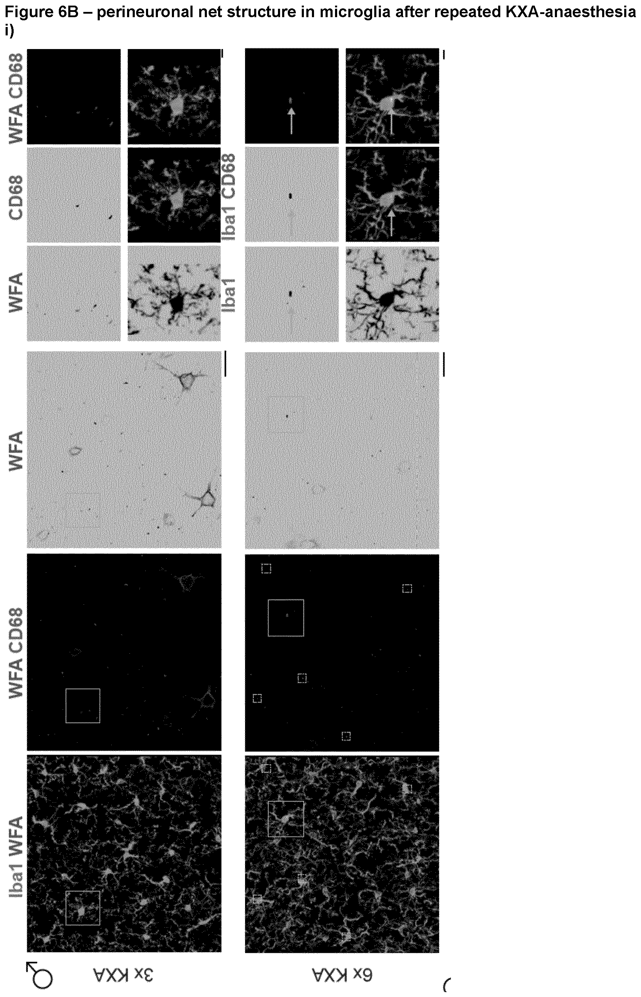

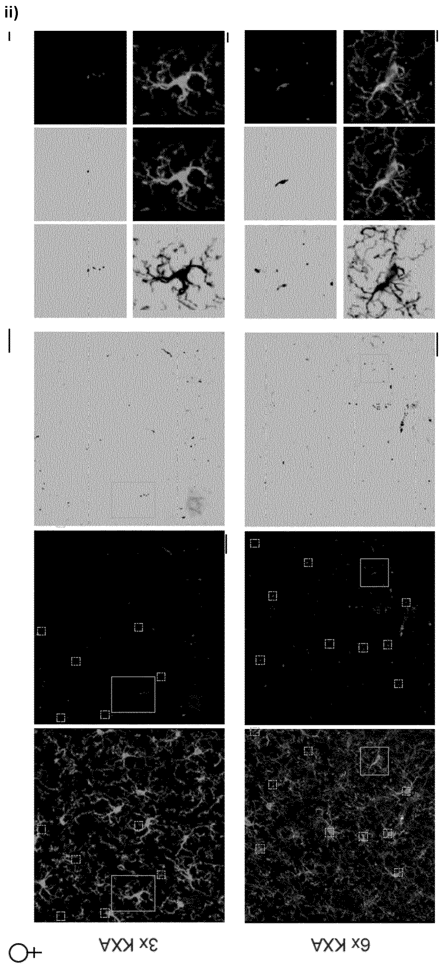

**Figure 6** - Microglia contain PNN fragments after repeated KXA-anesthesia in both sexes in primary somatosensory cortex (S1). Representative maximum intensity projection (MIP) overview images showing immunostained PNN (WFA, magenta), microglia (Iba1, green), and endosomal-lysosomal marker CD68 (blue) in S1 after 6x saline injection (control, **Figure 6A**) or 3x and 6x KXA (**Figure 6B**) for **(i)** male and **(ii)** female. Dashed frames: CD68/WFA colocalizations within field of view. Scale bar: 30 µm. Full frame: zoom-in. Scale bar: 5 µm.

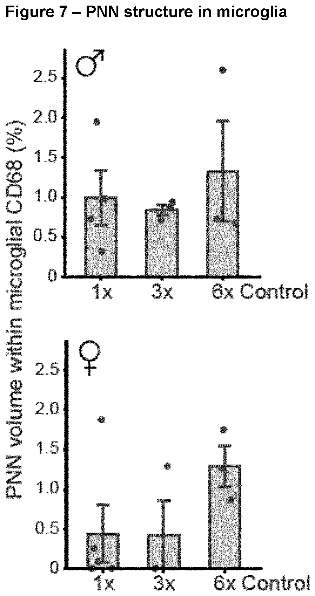

**Figure 7****:** Bar chart of PNN volume within microglia CD68 for repeated saline injection. 3-4 animals per condition. Linear regression model: p_{♂}=0.83. p_{♀}=0.17. KXA: ketamine-xylazine-acepromazine; PNN: perineuronal net; S1: primary somatosensory cortex; WFA: *Wisteria floribunda* agglutinin.

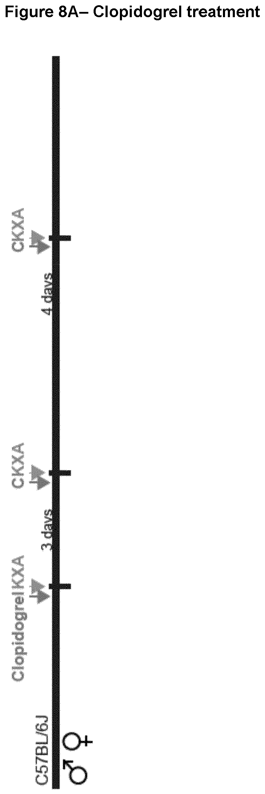

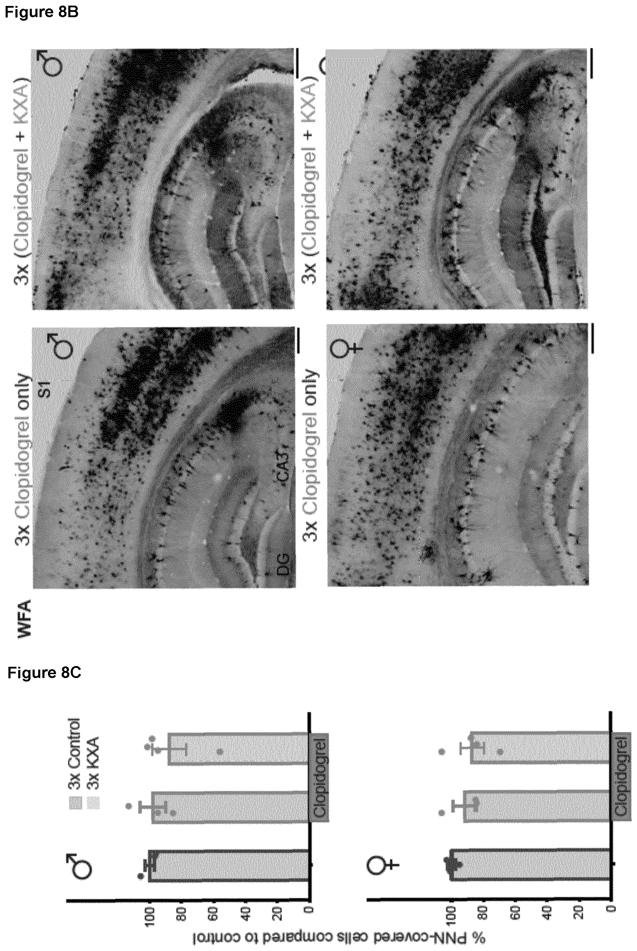

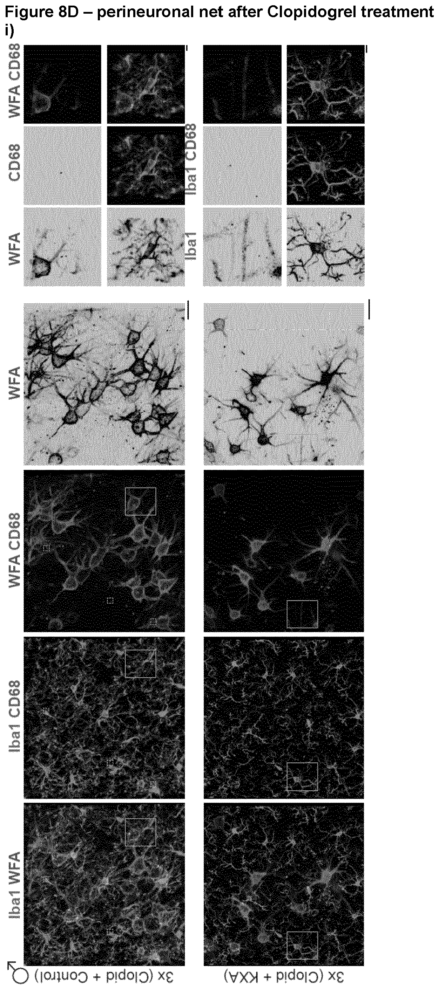

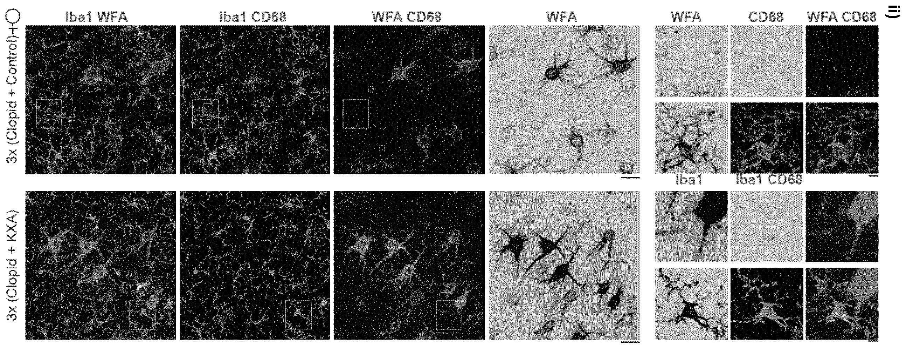

**Figure 8** - Clopidogrel prevents PNN removal by microglia upon KXA-anesthesia in the primary somatosensory cortex (S1). **Figure 8A****:** Experimental protocol. Clopidogrel (C) was injected *i.p.* 5 min before saline (Control) or KXA application. **Figure 8B****:** Overview images of PNN distribution stained with WFA (color inverted) for 3x Clopidogrel only or 3x (Clopidogrel+ KXA) in male (top) and female (bottom). Scale bar: 200 µm. **Figure 8C****:** Bar chart of per cent PNN-covered cells in the S1 for clopidogrel treatment for male (top) and female (bottom). 3-5 animals per condition. Linear regression model: p_{♂}=0.58. p_{♀}= 0.42. C, clopidogrel. CA3, *corno ammonis* region 3. DG, dentate gyrus. KXA, ketamine-xylazine-acepromazine. PNN, perineuronal net. S1, primary somatosensory cortex. **Figure 8D**: Representative maximum intensity projection (MIP) overview image showing immunostained PNN (WFA, *Wisteria floribunda* agglutinin, magenta), microglia (Iba1, green), and endosomal-lysosomal marker CD68 (blue) in S1 after 3 times clopidogrel and KXA anaesthesia (3x (Clopid+KXA)) or saline (Control). Dashed frames: CD68/WFA colocalizations within field of view. Scale bar: 30 µm. Full frame: zoom-in below. Scale bar: 5 µm.

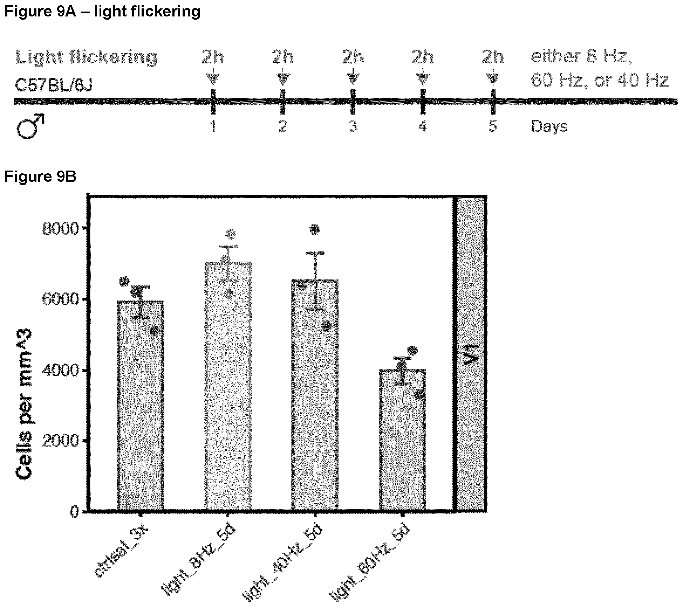

**Figure 9** - Light flickering stimulus triggers microglia to remove PNN in the primary visual cortex (V1). **Figure 9A****:** Experimental timeline for application of light flickering stimulus in C57BL6/J animals. **Figure 9B**: Density of PNN-covered cells in V1 after application of light flickering stimulus at different frequencies.

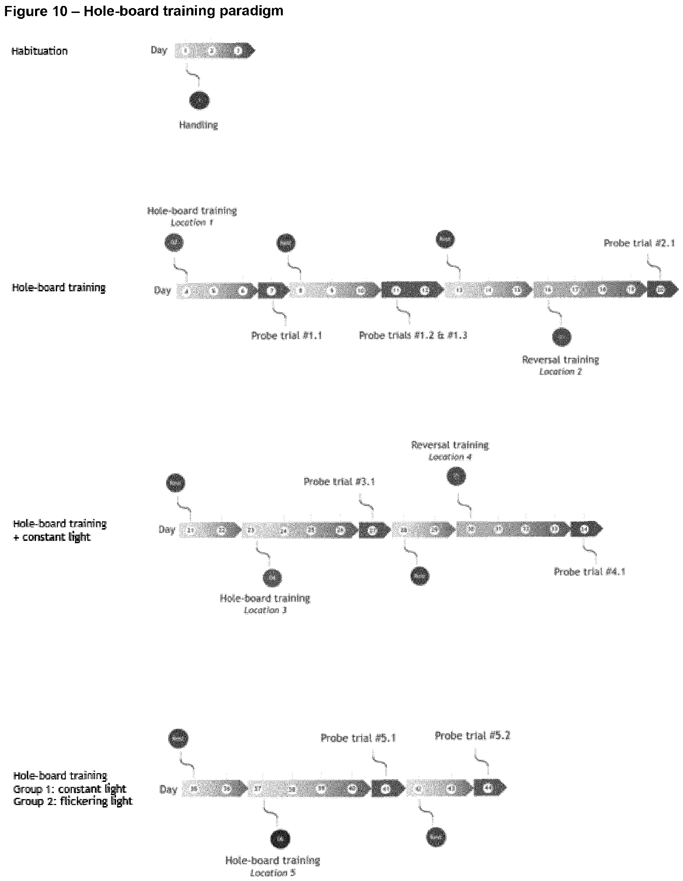

**Figure 10** - A timeline of the hole-board paradigm in Example 2.

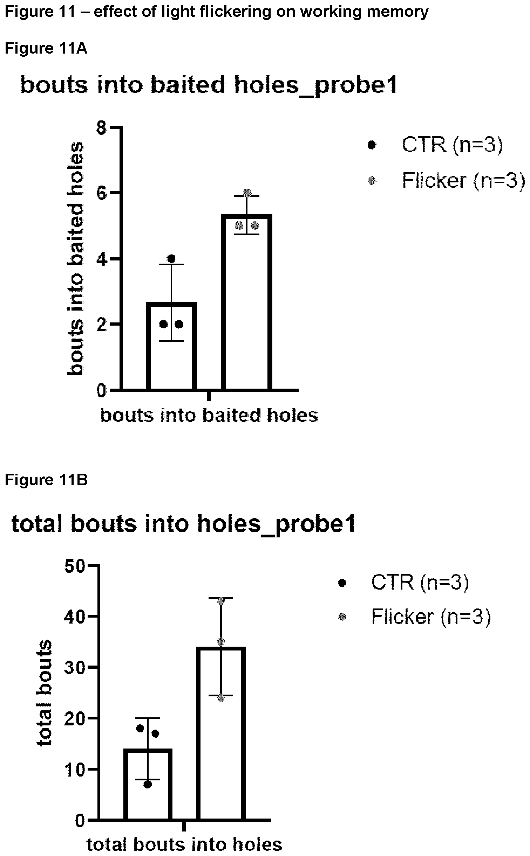

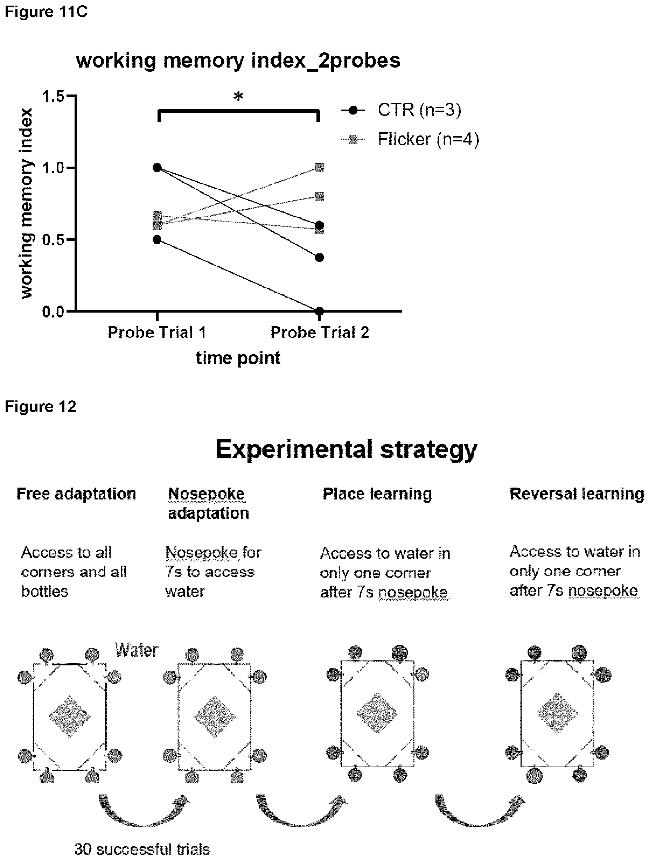

**Figure 11** - Effect of light flickering on working memory. **Figure 11A** - bouts into baited holes. **Figure 11B** - total bouts into holes. **Figure 11C** - improvement in working memory over time (probe trial 2 compared to probe trial 1).
**Figure 12** - Experimental strategy for Intellicage place learning and reversal learning studies in mice, as described in Example 1.

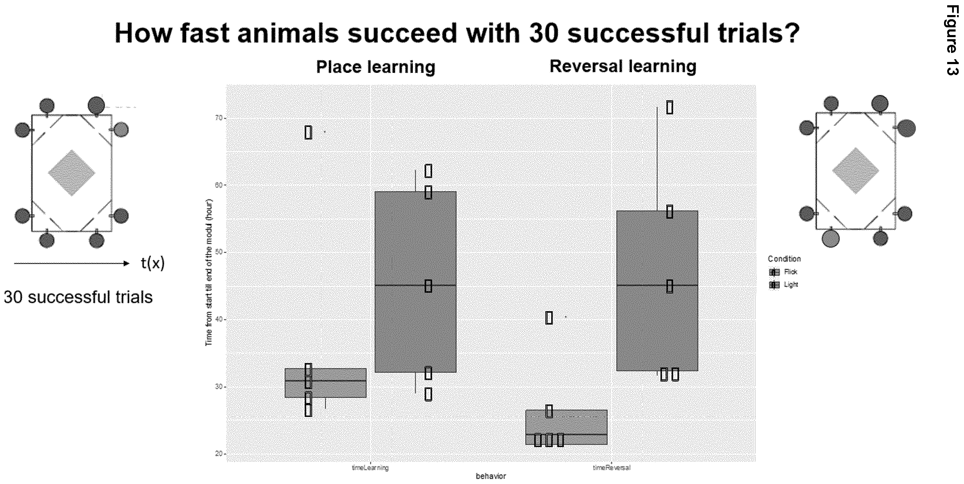

**Figure 13** - Time taken for 60 Hz light-treated ("Flick") and constant light-treated ("Light") mice to complete 30 successful trials in the place learning and reverse learning phases.

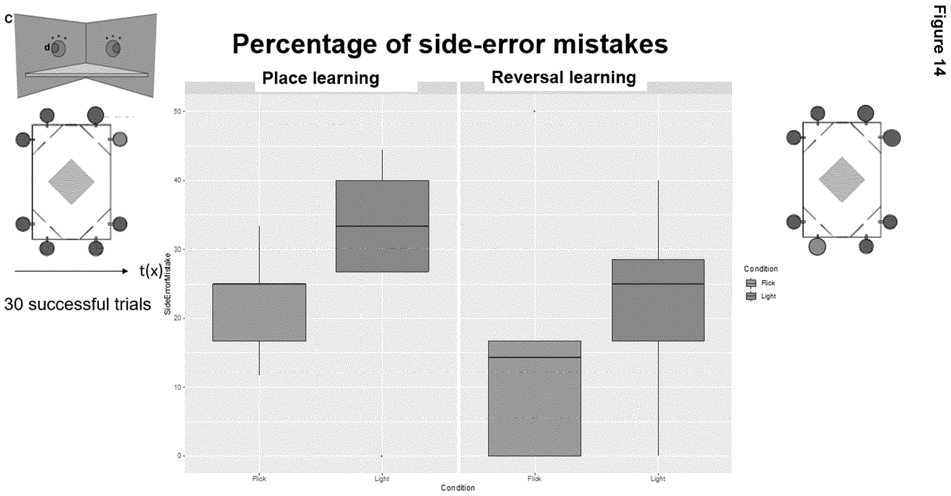

**Figure 14** - Percentage of side-error mistakes made by 60 Hz light-treated ("Flick") and constant light-treated ("Light") mice in the place learning and reverse learning phases.

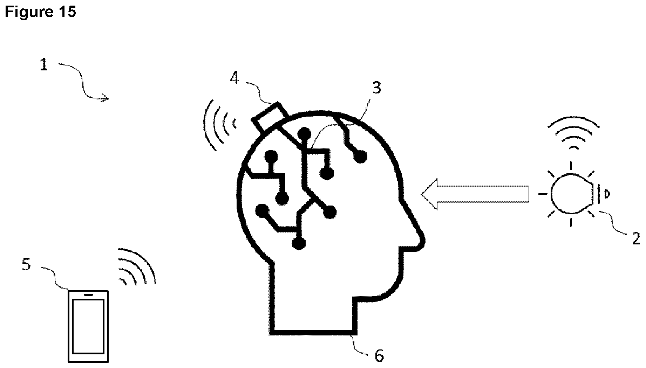

**Figure 15** - Schematic representation of one embodiment of a system for promoting neuronal plasticity in at least one brain region of a subject.

### DETAILED DESCRIPTION OF THE INVENTION

It is demonstrated herein that ketamine impacts PNN density and that repeated exposure to ketamine leads to PNN loss and re-opening of PNN-dependent plasticity. The key functional observations were changes in EEG oscillations, reduced network connectivity and cross frequency coupling, which might bridge the gap between abnormal signalling of GABA parvalbumin (PV)-positive interneurons and the cognitive deficit observed in the response seen in the mismatch negativity-like (MMN) component of event-related brain potentials (ERPs). This enables a new clinical context for the use of ketamine, e.g. to promote PNN loss and neuronal plasticity in the brain.

However, there is a strong desire to find alternatives to ketamine because its therapeutic use is not without dangers, not least because of its side effects and ease of misuse. Also, ketamine acts systemically across all CNS and brain regions. Therefore it is additionally demonstrated herein that a flashing light stimulus in the frequency range of about 50 to 70 Hz can replicate the effect of ketamine on PNN loss and plasticity. The present invention therefore additionally provides the use of such a flashing light stimulus to promote PNN loss and neuronal plasticity in the brain.

Abnormal oscillations in the gamma band (40-70 Hz frequency range) are considered to be a putative cause for cognitive deficits in schizophrenia and their first degree relatives. Robust reductions were found in auditory steady state potentials and oscillations around 40 Hz, which serves perception and cognition providing a mechanism for temporal binding of neural activities underlying mental representations (Cho R.Y. et al (2006) Proc. Natl. Acad. Sci. USA 103:19878-19883; Kwon J.S. et al (1999) Arch. Gen. Psychiatry 56:1001-1005). Converging lines of evidence implicate dysfunction in synchronisation of gamma network oscillations in the pathophysiology of schizophrenia.

The parvalbumin interneurons have a key role in the genesis of gamma oscillations in cortical networks as they exert a strong temporal inhibition onto their target pyramidal cells and interneurons (Whittington M.A. & Traub R.D. (2003) Trends Neurosci. 26:676-682; Sohal V.S. et al (2009) Nature 459:698-702.). Reduction or disturbance of NMDA receptor transmission on inhibitory GABAergic interneurons, which contain the PV-positive cells, may contribute to disturbances in gamma network dynamics and induce schizophrenia-like behaviour in animals (Uhlhaas P.J. & Singer W. (2010) Nat. Rev. Neurosci. 11:100-113; Gonzalez-Burgos G. & Lewis D.A. (2012) Schizophr. Bull. 38:950-957; Lewis D.A. et al (2005) Nat. Rev. Neurosci. 6:312-324).

GABAergic fast spiking interneurons maintain a balance of excitation and inhibition in the cortical network and those PV-positive interneurons have a critical role in the induction and maintenance of synchronous gamma network oscillations, required for working memory and cognitive information processing. Post-mortem studies in the brains of schizophrenic patients (Lewis *et al* (*supra*); Beasley C.L. & Reynolds G.P. (1997) Schizophr. Res. 24:349-355) and preclinical pharmacological studies with NMDA receptor blockers, as well as neurodevelopmental animal models of schizophrenia, have shown a consistent reduction in the number of PV-positive cells and spine density in the frontal cortex, nucleus accumbens and hippocampus (Kittelberger K. et al (2012) Brain Struct. Funct. 217:395-409; 83 Nakatani-Pawlak A. et al (2009) Biol. Pharm. Bull. 32:1576-1583; 84 Yang C. et al (2016) Psychiatry Res. 239:281-283).

Alteration of cortical excitation-inhibition balance is believed to significantly contribute to the pathophysiology of psychiatric disorders such as depression and schizophrenia. Ketamine has demonstrated robust fast-onset antidepressant efficacy in numerous clinical trials. In this context, the experimental results presented herein provide a strong rationale for the use of agents and methods that induce high gamma frequency oscillations in the brain to treat conditions such as schizophrenia and depression, e.g. by promoting neuronal plasticity.

### Promoting neuronal plasticity

In one embodiment, disclosed herein is a method for promoting neuronal plasticity in a subject. By "neuronal plasticity" it is generally meant to refer to the ability of the CNS (e.g. brain) to change structure and/or function, e.g. in response to a stimulus. Neuronal plasticity may involve e.g. the formation of new functional circuits and/or connections, and thus the term includes synaptic plasticity (i.e. the ability to form new or stronger synapses interconnecting neurons). The terms "brain plasticity", "neuroplasticity" or "neural plasticity" may be used interchangeably with "neuronal plasticity".

In some embodiments described herein, neuronal plasticity is promoted by removal of the perineuronal net (PNN). The PNN may be identified e.g. by staining with *Wisteria floribunda* agglutinin (WFA), as described in the Examples. By "removal" of the PNN, it is typically meant that the PNN is at least partially lost or decreased in one or more brain regions. For instance the absolute number and/or density of PNN-covered cells may be reduced (in subjects treated according to the present method) in at least one brain region, e.g. by at least 5%, 10%, 30% or 50% compared to a control (untreated subjects). Removal of the PNN may open a window of opportunity to induce changes and allow the brain to "learn", e.g. by permitting the formation of new functional circuits or connections.

The PNN shows widespread distribution in the brain and preferentially surrounds cortical parvalbumin-positive interneurons. Thus in some embodiments, the method may comprise inducing removal of the perineuronal net surrounding parvalbumin-positive interneurons, preferably cortical parvalbumin-positive interneurons.

PNN loss may be induced in one or more CNS (e.g. brain) regions in the subject, preferably at least in the cortex or a part thereof, more preferably in the sensory cortex or a part thereof. For instance, in particular embodiments, the PNN may be at least partially removed in the primary somatosensory cortex and/or the primary visual cortex of the subject. In other embodiments, PNN loss may be induced in the spinal cord or a part thereof.

In some embodiments, PNN removal is mediated via microglial cells. Microglial involvement in PNN removal in the method described herein may be monitored using known markers of glial cells (e.g. GFAP which labels astrocytes) and/or endosomal lysosomal markers (such as CD68), to detect phagocytic activity.

Preferably the PNN removal induced by methods described herein is temporary. For instance, the PNN may be removed for up to 1 week, up to 2 weeks, up to 4 weeks, up to 3 months, up to 6 months or up to 1 year, e.g. for 1 day to 6 months, 1 week to 3 months or 1 to 2 months. Without being bound by theory, temporary removal of the PNN may promote neuronal plasticity during the period of removal, but may also permit the PNN to reform in an improved configuration which is more permissive to plasticity. Such changes may therefore promote both short and long term improvements in neuronal plasticity.

### Inducing synchronized gamma oscillations

The present method may involve inducing synchronized gamma oscillations (or waves) in at least one CNS (e.g. brain) region of the subject. Such oscillations reflect neural network activity, and because they emerge from synaptic activity, they provide a direct link between the molecular properties of neurons and higher level, coherent brain activity. The terms "gamma oscillations" and "gamma waves" are used interchangeably herein.

The gamma oscillations may be induced in a cell-type or CNS (e.g. brain)-region specific manner. For instance, the oscillations may be induced in parvalbumin-positive interneurons. In particular embodiments, the oscillations may be induced in the cortex, more preferably in the sensory cortex, and most preferably at least in the primary somatosensory cortex and/or the primary visual cortex of the subject.

Typically the gamma oscillations are high gamma frequency oscillations, e.g. in the region of 50 to 90 Hz. The gamma oscillations preferably have a frequency of about 50 to about 70 Hz. In particular embodiments the oscillations have a frequency of at least 51 Hz, at least 52 Hz, at least 55 Hz, at least 57 Hz, or at least 58 Hz. In other embodiments the oscillations have a frequency of up to 70 Hz, up to 68 Hz, up to 65 Hz or up to 63 Hz. Thus suitable preferred frequency ranges include e.g. 51 to 70 Hz, 53 to 70 Hz, 55 to 70 Hz, 55 to 65 Hz and 57 to 63 Hz. Most preferably the gamma oscillations have a frequency of about 60 Hz.

In general terms, gamma oscillations may be induced in the brain using methods, devices and systems analogous to those described in e.g. WO 2017/091698 and WO 2019/075094, except that according to the present disclosure, the oscillations are in the high gamma frequency range (e.g. 50 to 70 Hz). Thus in one embodiment, the present method includes the steps of controlling a stimulus-emitting device to emit a stimulus and exposing the subject to the stimulus and/or administering the stimulus to the subject, thereby inducing *in vivo* synchronized high gamma oscillations in at least one brain region of the subject. The stimulus-emitting device may be a haptic device, a light-emitting device, and/or a sound-emitting device. For example, the light-emitting device may be a fiber optic device.

A stimulus may include any detectable change in the internal or external environment of the subject that directly or ultimately induces high gamma oscillations in at least one brain region. For example, a stimulus may be designed to stimulate electromagnetic radiation receptors (e.g., photoreceptors, infrared receptors, and/or ultraviolet receptors), mechanoreceptors (e.g., mechanical stress and/or strain), nociceptors (i.e., pain), sound receptors, electroreceptors (e.g., electric fields), magnetoreceptors (e.g., magnetic fields), hydroreceptors, chemoreceptors, thermoreceptors, osmoreceptors, and/or proprioceptors (i.e., sense of position). The absolute threshold or the minimum amount of sensation needed to elicit a response from receptors may vary based on the type of stimulus and the subject. In some embodiments, a stimulus is adapted based on individual sensitivity.

By way of example, in some embodiments, the high gamma oscillations are induced in the visual cortex using a flashing light; and in other embodiments, the high gamma oscillations are induced in the auditory cortex using auditory stimulation at particular frequencies. In some embodiments, the high gamma oscillations are induced in multiple brain regions simultaneously using a combination of visual, auditory, and/or other stimulations. In some embodiments, the high gamma oscillations are induced in a virtual reality system.

In some embodiments, the subject receives a stimulus via an environment configured to induce high gamma oscillations, such as a chamber that passively or actively blocks unrelated stimuli (e.g., light blocking or noise canceling). Alternatively or in addition, the subject may receive a stimulus via a system that includes, for example, light blocking or noise canceling aspects. In some embodiments, the subject receives a visual stimulus via a stimulus-emitting device, such as eyewear designed to deliver the stimulus. The device may block out other light. In some embodiments, the subject receives an auditory stimulus via a stimulus- emitting device, such as headphones designed to deliver the stimulus. The device may cancel out other noise.

In one embodiment, the high gamma oscillations are induced by a visual stimulus, e.g. a flashing light. The terms "flashing" and "flickering" are used herein interchangeably, in general to refer to an intermittent or pulsing light source. In particular embodiments, the subject may be exposed to a plurality of light pulses. The light pulses (and thus the flashing light) may, for example have a frequency equivalent to that of the desired gamma oscillations. Thus the light pulses may have a frequency of at least 51 Hz, at least 52 Hz, at least 55 Hz, at least 57 Hz, or at least 58 Hz. In other embodiments the light pulses have a frequency of up to 70 Hz, up to 68 Hz, up to 65 Hz or up to 63 Hz. Suitable preferred frequency ranges for the light pulses include e.g. about 50 to about 70 Hz, 51 to 70 Hz, 53 to 70 Hz, 55 to 70 Hz, 55 to 65 Hz and 57 to 63 Hz. Most preferably the light pulses have a frequency of about 60 Hz. The subject may, for example, be placed in a chamber to which such light pulses are applied, or may wear a light-blocking device that emits suitable light pulses.

Each light pulse may, for example, have a duration of less than 20, less than 15 or less than 10 milliseconds, e.g. about 1 ms, about 5 ms or about 8 ms. For instance the light pulses may have a duration of about 5 to 12 ms or about 6 to 10 ms. In one embodiment the light pulses have a frequency of about 60 Hz, each light pulse has a duration of about 8.3 ms and the light pulses are separated by an interval of about 8.3 ms (i.e. a dark interval of 8.3 ms with no light).

The light pulses are typically in the visible range, e.g. having a wavelength of about 380 to 740 nanometers, e.g. about 470 nm. The light can be of any colour, but is preferably white light. For the avoidance of doubt, when referring to "frequency" of light herein it is of course meant the frequency of the pulses of light, and not the electromagnetic frequency of light itself.

In general, any suitable light intensity may be used. However in a preferred embodiment, the light has an intensity of about 1 x 10¹⁸ to x 10¹⁹ photons/cm²/s, e.g. 2 - 6 x 10¹⁸ photons/cm²/s, for example about 4 x 10¹⁸ photons/cm²/s. The power and electromagnetic frequency of the light can be selected to achieve the desired light intensity.

The light source may be a single point of light or an array of light sources. In some embodiments, the visual stimulus may be generated by a light-emitting diode (LED), or an array of LEDs. It should be appreciated, however, that various types of devices may be employed other than LED-based devices to effectively deliver the visual stimulus at various frequencies within the ranges defined herein. In some embodiments, an array of LEDs with a square wave current signal may be employed. LEDs are preferably white LEDs but can be any other LED colour. Further methods, systems and devices, which may be suitably modified, adapted or configured to deliver the stimulus at a high gamma frequency (e.g. 50 to 70 Hz), are described in WO 2018/094226 and WO 2018/094232.

In other embodiments, the stimulus may be generated by a sound source, e.g. configured to generate auditory pulses in a similar frequency to that described above light pulses. For instance, the device may comprise an electroacoustic transducer to convert an electrical audio signal into a corresponding sound stimulus. Thus the sound source may generate e.g. a click train with a click frequency of e.g. 50 to 70 Hz, 51 to 70 Hz, 53 to 70 Hz, 55 to 70 Hz, 55 to 65 Hz and 57 to 63 Hz. Most preferably the clicks have a frequency of about 60 Hz. In embodiments comprising both a light and sound stimulus, it will be appreciated that the sound may be emitted at the same frequency or at a different frequency to the light.

Each click in the click train may preferably have a duration of less than 10 ms, e.g. about 1 ms. Each click in the click train may have a sound pressure level of e.g. about 1 dB to about 85 dB, about 30 dB to about 70 dB, or about 60 dB to about 65 dB. Alternatively or in addition, the sound may be emitted at a volume that varies over a selected period of time.

The at least one electroacoustic transducer may include at least one headphone, in which case the method may include applying the at least one headphone around, on, and/or in at least one ear of the subject to direct the sound stimulus into the at least one ear of the subject. The method also may include reducing ambient noise using passive noise isolation and/or active noise cancellation.

The sound source may be incorporated into the same device as the light source, and or may be operated independently. In general, it will be appreciated that the devices, functions and components used in the present method may be embodied in a system comprising independent component parts, or may be implemented in a single device. In addition to at least one interface for emitting a stimulus, some embodiments of the device or system may include at least one processor (to, e.g., generate a stimulus, control emission of the stimulus, monitor emission of the stimulus/results, and/or process feedback regarding the stimulus/results), at least one memory (to store, e.g., processor-executable instructions, at least one stimulus, a stimulus generation policy, feedback, and/or results), at least one communication interface (to communicate with, e.g., the subject, a healthcare provider, a caretaker, a clinical research investigator, a database, a monitoring application, etc.), and/or a detection device (to detect and provide feedback regarding, e.g., the stimulus and/or the subject, including whether gamma oscillations are induced, subject sensitivity, cognitive function, physical or chemical changes, stress, safety, etc.).

The system or device may thus further comprise a timer connected to the stimulus source, e.g. light source. The timer enables the light source to emit light for a selected period of time. In particular embodiments the stimulus (e.g. light) is emitted for at least e.g. 1 min, 5 mins, 10 mins, 20 mins, 30 minutes, 1 hour, 1.5 hours or 2 hours, preferably for at least 1 hour, at least 1.5 hours or at least 2 hours, more preferably 1 to 3 hours. The duration of the exposure of the subject to the stimulus and/or the administration of the stimulus to the subject may be e.g. 1 to 3 hours, for instance about one hour. The exposure of the subject to the stimulus and/or the administration of the stimulus to the subject may be repeated over a time period. For example, the exposure of the subject to the stimulus and/or the administration of the stimulus to the subject may be repeated at least once per day over the time period. The time period may include, but is not limited to, 1 day, 2 days, 3 days, 4 days, 5 days, 6 days, one week, two weeks, three weeks, and/or one month (or longer, such as once daily for the rest of the subject's life). In a particular example, the period of time is 2 hours for 5 consecutive days.

The systems, devices and methods described herein may advantageously provide improvements in cognitive function and alleviation of neuropsychiatric disorders following a relatively short treatment time. For instance, as demonstrated in Example 2 below, mice exposed to only 5 minutes daily of 60Hz flickering light (or 5 minutes during a learning task and 5 minutes afterwards, total 10 minutes) showed significant improvements in cognitive function (e.g. in working memory, memory preservation and retrieval, see Figures 11A-C). Thus in preferred embodiments, a subject may be exposed to the stimulus (e.g. 60 Hz flickering light) for less than 1 hour, e.g. 10 seconds to 50 minutes, 30 seconds to 30 minutes, 1 minute to 10 minutes, 2 to 8 minutes or about 5 minutes. This duration of exposure may be repeated e.g. up to 4 times daily, e.g. once or twice daily, or 1 to 6 times a week (e.g. once, twice or three times a week). The treatment may continue for e.g. one week, two weeks, three weeks, and/or one month (or longer. In particularly preferred embodiments, the subject is exposed to the stimulus (e.g. light at 60 Hz) for 1 to 10 minutes once daily for one week or more.

Preferably the stimulus (e.g. light) is administered to the subject whilst the subject is awake, e.g. whilst the subject's eyes are open. In other embodiments, the stimulus (e.g. light) may be administered whilst the subject's eyes are closed and/or is asleep.

In some embodiments, the system or device may further include means to vary light intensity. In this way, the light intensity may be varied over a selected period of time. In preferred embodiments, treatment may be applied in a closed system such that is substantially free from ambient light.

A further method for induction of gamma oscillations includes non-invasive brain stimulation (NIBS), e.g. as an adjunct to light pulses. NIBS includes repetitive Transcranial Magnetic Stimulation (rTMS), transcranial Direct Current Stimulation (tDCS) and transcranial Alternating Current Stimulation (tACS). Suitable methods are reviewed, for example, in Wagner et al., Ann. Rev. Biomed. Eng. (2007); 9:527-65.

Entrainment of high frequency gamma oscillations in the brain can be confirmed using various methods. For instance, oscillations in the gamma frequency band of the electroencephalogram (EEG) may be monitored. EEG analysis in response to a particular treatment may be performed using methods as described in e.g. Ahnaou et al., Neuropharmacology (2014); 86:362-377 or Castro-Zaballa et al., Frontiers in Psychiatry (Jan 2019) 9:Article 766. Another suitable method for assessing gamma oscillations involves magnetoencephalography using the auditory steady state response, e.g. as described in Tsuchimoto et al., Schizophr Res. (Dec 2011); 133(1-3):99-105.

Thus in some embodiments, the method may involve induction of gamma wave entrainment and optionally measuring or monitoring gamma wave entrainment in response thereto. Monitoring of gamma wave entrainment may be performed in one or more brain regions of interest. By "induction of gamma wave entrainment" it is meant that the brain of the subject is induced to enter a state in which gamma waves are produced in the relevant frequency band (i.e. about 50 to 70 Hz), e.g. by a sensory stimulus in a corresponding frequency band. The brain may typically adopt a "frequency following" response, in which oscillations in neuronal activity align to the frequency of the stimulus.

Thus gamma wave entrainment may be monitored by methods such as EEG analysis, and entrainment of gamma waves in the 50 to 70 Hz band in one more brain regions may be indicative of successful entrainment. The monitoring device (e.g. EEG apparatus) may be incorporated in a system comprising the stimulus-emitting device, e.g. in the same device or in separate devices that are functionally linked. For instance, in some embodiments a sensory (e.g. visual) stimulus may be continued until gamma wave entrainment has been successfully achieved for a predetermined time period, as determined by the monitoring. The predetermined period of time may be e.g. at least 10 mins, 30 minutes, 1 hour, 2 hours or 3 hours. Thus the monitoring device (e.g. EEG apparatus) may provide feedback to the stimulus-emitting device in order to control e.g. the duration, frequency and/or intensity of the stimulus (e.g. light pulses).

Therefore in some embodiments, the duration or intensity of flickering light is adjusted for each subject on a personalized basis. In one embodiment, the system comprises a stimulus-emitting device (e.g. a wearable LED source) and a monitoring device (e.g. EEG apparatus) connected to a microprocessor. The components of the system may be embodied in the same device or in separate linked devices. The system may utilize any suitable radio communication method (e.g. Bluetooth) to communicate with an external user interface, e.g. a mobile device operating a suitable app. The monitoring apparatus may constantly track and analyze the brain activity of the subject and provide a real-time neurofeedback to the stimulus emitting device. The system may thus modulate the intensity and/or duration of the light provided by the stimulus-emitting device and automatically turn on or off the device when is needed. A user (e.g. the subject) can interact with the device and monitor his brain activity directly from the user interface (e.g. a smartphone or other mobile device) and, if needed, show the EEG recording to a clinician.

One suitable embodiment is shown in Figure 15. The system (1) comprises a light source (2), a monitoring or detection device (3), a processor (4) and a user or communication interface (5). The light source 2 is configured to direct flashing light at 50-70 Hz (e.g. 60 Hz) towards the eyes of a subject (6). The light source may be e.g. a series of LEDs, which may be mounted in a suitable headset for directing light towards the eyes. The monitoring device 3 (e.g. an EEG apparatus) detects brain activity in the subject via a series of electrodes. The detected brain activity may be transmitted to the user interface 5 for display, via a suitable communications protocol or network (e.g. Bluetooth). The user interface 5 may also communicate with e.g. the light source 2, e.g. to allow a user (e.g. the subject 6 or a clinician) to set parameters such as the duration, intensity and frequency of the emitted light.

The monitoring device 3 is connected to the processor 4, which analyses the detected brain activity, in particular to identify synchronized gamma oscillations at 50-70 Hz in the brain of the subject. The processor 4 may communicate with the light source 2 to modulate the intensity, frequency or duration of the light emitted by the light source 1, based on e.g. whether synchronized gamma oscillations at 50-70 Hz are detected in the subject's brain. For instance if synchronized gamma oscillations at 50-70 Hz are not detected (or only weak signals are detected), the processor 4 may increase the intensity of the light emitted by the light source 2. Alternatively the processor 4 may modulate the frequency of emitted light, e.g. by 1 or 2 Hz within the 50-70 Hz frequency band, in order to identify a suitable frequency for generating synchronized oscillations. If the processor 4 determines that synchronized gamma oscillations at 50-70 Hz have been detected for a sufficient period of time (e.g. 5 minutes), the processor 4 may switch off the light source 2.

It will be appreciated that various wired and wireless arrangements of the system 1 shown in Figure 15 are contemplated. For instance, the light source 2 and monitoring device 3 may be embodied in a single physical device or in separate device communicating via a wireless network (e.g. Bluetooth). Similarly the processor 4 may be provided within the monitoring device 3, the light source 2 or the user interface 5. The user interface 5 may also be present within a single device comprising the monitoring device 3 and/or light source 2.

### Pharmaceutical compositions

In embodiments as discussed above, a stimulus such as flashing light (or sound) is applied to the subject in order to induce the high gamma frequency neural oscillations. In alternative embodiments, a pharmaceutical composition comprising ketamine may be administered to the subject in order to achieve a similar effect. The pharmaceutical composition may comprise ketamine and optionally one or more pharmaceutically acceptable carriers, diluents and/or excipients.

Ketamine (i.e. 2-(2-chlorophenyl)-2-(methylamino)cyclohexan-1-one) is a well-known non-competitive N-methyl- D-aspartate (NMDA) receptor antagonist. Ketamine is optically active and may be administered as a racemic mixture (i.e. *RS*-ketamine) or as the more active enantiomer, esketamine (*S*-ketamine). In alternative embodiments, the ketamine may be *R-*ketamine. Ketamine may be formulated and administered to a subject e.g. as described in publications such as WO2007/111880. Such a treatment may be administered alone or may be supplemented with other antipsychotic or antidepressant therapies. Ketamine may be administered intramuscularly (i.m.), intravenously (i.v.), intranasally or via caudal, intrathecal, and subcutaneous (s.c) routes.

A suitable dose of ketamine may be e.g. approximately 0.01 to approximately 1 mg/kg of body weight. In a more preferred aspect, the dose of ketamine is approximately 0.05 to approximately 0.7 mg/kg of body weight. In another embodiment, the total dose of ketamine per nasal administration ranges from about 1 to about 250 mg. The actual dose will vary, depending on the body weight of the patient, the severity of the condition, the route of administration, the nature of medications administered concurrently, the number of doses to be administered per day, and other factors generally considered by the ordinary skilled physician in the administration of drugs.

In a specific embodiment, the amount of ketamine administered to a patient is about 10% to about 20% of the amount used to induce anesthesia. Alternatively ketamine may be administered in a higher dose, e.g. in an amount sufficient to induce anaesthesia. In another specific embodiment, the dose of ketamine is about 0.01 mg per kg of body weight (0.01 mg/kg) to about 1 mg/kg; preferably about 0.05 mg/kg to about 0.7 mg/kg. In yet another embodiment, the dose ranges from about 1 mg to about 250 mg. A dose of any integer between these two numbers is contemplated. Thus, for example, intranasal, transdermal, intravenous, intradermal, or subcutaneous formulations respectively containing total intranasal, transdermal, intravenous, intradermal, or subcutaneous doses of 1 mg, 2 mg, 4 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg are specifically contemplated. Preferably, the effective dose is titrated under the supervision of a physician or medical care provider, so that the optimum dose for the particular application is accurately determined. Thus, the present invention provides a dose suited to each individual patient.

Ketamine is formulated into pharmaceutical compositions comprising a carrier suitable for the desired delivery method. Exemplary carriers include, but are not limited to, any of a number of standard pharmaceutical carriers such as sterile phosphate buffered saline solutions, bacteriostatic water, and the like. A variety of aqueous carriers maybe used, e.g., water, buffered water, 0.4% saline, 0.3% glycine and the like.

In some embodiments, ketamine may be administered via a nasal inhaler containing an aerosol formulation of ketamine and a pharmaceutically acceptable dispersant. The dispersant may be a surfactant, such as, but not limited to, polyoxyethylene fatty acid esters, poiyoxyethylene fatty acid alcohols, and polyeoxyethylene sorbitan fatty acid esters. Phospholipid-based surfactants also may be used.

In other embodiments, the aerosol formulation of ketamine is provided as a dry powder aerosol formulation in which the ketamine is present as a finely divided powder. The dry powder formulation can further comprise a bulking agent, such as, but not limited to, lactose, sorbitol, sucrose and mannitol.

In another specific embodiment, the aerosol formulation is a liquid aerosol formulation further comprising a pharmaceutically acceptable diluent, such as, but not limited to, sterile water, saline, buffered saline and dextrose solution. The formulation may include a carrier. The carrier is a macromolecule which is soluble in the circulatory system and which is physiologically acceptable where physiological acceptance means that those of skill in the art would accept injection of said carrier into a patient as part of a therapeutic regime. The carrier preferably is relatively stable in the circulatory system with an acceptable plasma half life for clearance. Such macromolecules include but are not limited to Soya lecithin, oleic acid and sorbitan trioleate, with sorbitan trioleate preferred.

In another embodiment, the pharmaceutical formulation may comprise a transdermal patch containing ketamine and a pharmaceutically acceptable carrier.

The pharmaceutical composition may be designed to be short-acting, fast- releasing, long-acting, or sustained-releasing as described herein. Thus, the pharmaceutical formulations may also be formulated for controlled release or for slow release.

In further embodiments, the pharmaceutical formulation can include other therapeutically or pharmacologically active ingredients in addition to ketamine, such as but not limited to a conventional antidepressant therapies that include, but are not limited to: antidepressants: biogenic amine non-selective reuptake inhibitors, e.g., tricyclic antidepressants like Imipramine; serotonin selective reuptake inhibitors like Fluoxetine (Prozac); monoamine oxidase inhibitors (MAO-I) like phenelzine; other types of antidepressant medications including atypical antidepressants.

### Treatment of neurological and/or neuropsychiatric disorders

The methods, devices and systems (including e.g. pharmaceutical compositions comprising ketamine) described herein may be used in general for:
(i) inducing synchronized gamma oscillations in at least one brain region of a subject; wherein the synchronized gamma oscillations have a frequency of about 50 to about 70 Hz;
(ii) promoting removal of the perineuronal net in a brain region of a subject; and/or
(iii) promoting neuronal plasticity in a brain region of a subject.

Typically the methods, devices and systems are applied for the prevention or treatment of a neurological, psychiatric, neurodevelopmental or neurodegenerative disorder. Particular examples of psychiatric disorders are affective, cognitive, psychotic or behavioural disorders, preferably schizophrenia, bipolar disorder or depression. In further embodiments, the disorder to be treated may be anxiety, obsessive compulsive disorder (OCD), eating disorders, post-traumatic stress disorder (PTSD), drug addiction, pain, autistic spectrum disorder, dementia or a sleep disorder. The disorder may alternatively be a traumatic injury, e.g. a brain or spinal cord injury. In particular, the disorder can be one which is associated with learning and/or memory deficits, such as depression, anxiety, PTSD and OCD. These deficits can be identified by standardized neuropsychological testing.

The present invention provides a new clinical frame in which e.g. ketamine or light pulses may be used to treat such conditions via promotion of neuronal plasticity. The methods, devices and systems described herein may therefore be used to treat e.g. a sub-group of patients suffering from such conditions in which it is desirable to induce high gamma frequency oscillations, to remove the PNN and/or to promote neuronal plasticity. Other patient subgroups to be treated include subjects who are undergoing concurrent treatment with neuropsychotropic drugs, such as antidepressants, anti-anxiety, anti-psychotic, mood stabilizing, and stimulant medications.

Types of depression that may be treated include but are not limited to any of: major depressive disorder, single episode, recurrent major depressive disorder-unipolar depression, seasonal affective disorder- winter depression, bipolar mood disorder-bipolar depression, mood disorder due to a general medical condition-with major depressive-like episode, or mood disorder due to a general medical condition-with depressive features, including where those disorders are resistant to treatment in a given patient. In a preferred embodiment, the present invention is used to treat a subject with treatment-resistant depression.

There are three types of depression generally characterized in the art, major depression, dysthymic disorder, or dysthymia, and depressive disorder not otherwise specified. Major depression is characterized by peak episodes of extreme depression. During a peak episode, the patient may suffer from depressed mood, and markedly diminished interest or pleasure in activities. Other symptoms include significant weight loss or weight gain, decrease or increase in appetite, insomnia or hypersomnia, psychomotor agitation or retardation, fatigue or loss of energy, feelings of worthlessness or excessive or inappropriate guilt, diminished ability to think or concentrate or indecisiveness, recurrent thoughts of death, suicidal ideation or suicidal attempts. Symptoms last for at least two weeks and cause significant distressor impairment in important areas of functioning.

Dysthymia is characterized by depressed mood for at least 2 years as well as other symptoms like poor appetite or overeating, insomnia or hypersomnia, low energy or fatigue, low self esteem, poor concentration or difficulty making decisions and feelings of hopelessness. As is recognized in the field of psychiatric arts, depression may also comprise, and/or may also manifest itself in a variety of forms, including but not limited to, seasonal affective disorder, diurnal mood variations, or depression associated with menopause. Diagnostic criteria for dysthymia and major depression, as well as for seasonal affective disorder, diurnal mood variations and depression associated with menopause, are more fully explained in the Diagnostic and Statistical Manual of Mental Disorders, Fourth Edition, (DSM IV) published by the American Psychiatric Association or by the ICD (ICD-10: International Statistical Classification of Diseases and. Related Health Problems, 10th Revision) or any other psychiatric classification system.

Depression with seasonal affective pattern or seasonal affective disorder (hereinafter referred to as "SAD") is also known as cabin fever, evening blues, and sun deprivation syndrome. The terms "seasonal affective disorder" or "seasonal pattern specifier" are defined in the DSM-IV as a specifier or adjective that more precisely characterize feature associated with depression. A particular feature of SAD is the regular occurrence of depression in winter. Most of the patients with SAD are characterized by an atypical type of depression in the winter which is associated with mood reactivity (mood brightens in response to actual or potential positive events) as well as weight gain or increase in appetite, hypersomnia, leaden paralysis (heavy, leaden feelings in arms or legs), long-standing pattern of interpersonal rejection sensitivity.

Psychotic conditions such as schizophrenia and related disorders, for example schizoaffective disorder, are complex and heterogeneous diseases of uncertain etiology. With a worldwide prevalence of approximately one percent to two percent of the population, schizophrenia has serious social and economic consequences.

Schizophrenia itself is characterized by fundamental distortions in realms of thinking and perception, cognition and the experience of emotions. With a typical onset in late adolescence or early adulthood, it is a chronic lifelong illness with periods of frank psychotic features alternating with periods of residual symptoms and incomplete social recovery. Schizophrenia requires medical intervention in virtually all cases. Approximately 60% to 70% of schizophrenic patients never marry and the unemployment rate among schizophrenic patients is greater than 70%. Such statistics suggest that schizophrenic patients do not adequately function in society.

Symptoms of schizophrenia are subdivided into three major clusters: positive, negative, and cognitive. Positive (psychotic) symptoms, consist of delusions (false beliefs that cannot be corrected by reason), hallucinations (usually nonexistent voices), disorganized speech, and grossly disorganized behavior. Negative symptoms are described as affective flattening, alogia (speechlessness caused by mental confusion), avolition (lack of motivation to pursue a goal), and anhedonia (inability to experience pleasure). Cognitive deficits include impairments of working memory, attention, verbal reproduction, and executive function. Furthermore, a variety of associative features and mental disorders include poor insight, depersonalization, derealization, depression, anxiety, and substance abuse disorders. Finally, schizophrenia patients have a markedly increased risk of suicide rate with 20% to 40% attempting suicide at least once in their lifetime, and 10% of patients successively committing suicide.

The methods, devices and systems described herein may thus be used to treat psychotic disorders, including schizophrenia. Examples of psychotic disorders that can be treated according to the present invention include, but are not limited to, schizophrenia, for example of the paranoid, disorganized, catatonic, undifferentiated, or residual type; schizophreniform disorder; schizoaffective disorder, for example of the delusional type or the depressive type; delusional disorder; brief psychotic disorder; shared psychotic disorder; psychotic disorder due to a general medical condition; substance-induced psychotic disorder, for example psychosis induced by alcohol, amphetamine, cannabis, cocaine, hallucinogens, inhalants, opioids, or phencyclidine; personality disorder of the paranoid type; personality disorder of the schizoid type; psychotic disorder not otherwise specified.

The meanings attributed to the different types and subtypes of psychotic disorders are as stated in DSM-IV-TR. (Diagnostic and Statistical Manual of Mental Disorders, 4th ed., American Psychiatric Assoc., Washington, D.C., 2002, p. 297-343).

Schizophrenia as used herein refers to a disorder that lasts for at least 6 months and includes at least one month of active-phase symptoms (i.e., two [or more] of the following: delusions, hallucinations, disorganized speech, grossly disorganized or catatonic behavior, negative symptoms).

Schizoaffective disorder is defined as a disorder in which a mood episode and the active-phase symptoms of schizophrenia occur together and were preceded or are followed by at least 2 weeks of delusions or hallucinations without prominent mood symptoms.

Schizophreniform disorder is defined as a disorder characterized by a symptomatic presentation that is equivalent to schizophrenia except for its duration (i.e., the disturbance lasts from 1 to 6 months) and the absence of a requirement that there be a decline in functioning.

Schizotypical disorder is defined as a lifetime pattern of social and interpersonal deficits characterized by an inability to form close interpersonal relationships, eccentric behavior, and mild perceptual distortions.

Post-Traumatic stress disorder (PTSD) is a disorder that develops in some people who have experienced a shocking, scary, or dangerous event. It is natural to feel afraid during and after a traumatic situation. Fear triggers many split-second changes in the body to help defend against danger or to avoid it. This "fight-or-flight" response is a typical reaction meant to protect a person from harm. Nearly everyone will experience a range of reactions after trauma, yet most people recover from initial symptoms naturally. Those who continue to experience problems may be diagnosed with PTSD. People who have PTSD may feel stressed or frightened, even when they are not in danger (NIH Definition). PTSD may be associated with aberrant synaptic plasticity, as described in e.g. Holmes et al. Nature Communications (2019); Volume 10, Article number: 1529.

Anxiety disorders are extremely common, with a lifetime prevalence of between 5 and 30% in the general population. Patients suffering from anxiety disorders experience excessive worry or fear and often have associated physical symptoms. Examples of anxiety disorders include Post-traumatic stress disorder (PTSD), Generalized anxiety disorder (GAD), social anxiety disorder, panic disorder, and phobias.

Obsessive Compulsive Disorder (OCD) is a chronic condition characterized by uncontrollable and distressing thoughts (obsessions) of diverse natures, such as contamination, need for symmetry, intrusive thoughts, or rumination, coupled with compulsions that are repetitive behaviours such as hand-washing, checking, counting, and reassurance-seeking. Some OCD patients can benefit from pharmacologically therapies such as antidepressants at high doses but even with psychotherapy many struggle to break out of patterns of behavior that severely impact on their quality of life. Imaging studies have shown differences in the frontal cortex and subcortical structures of the brain in patients with OCD.

In a preferred embodiment, the patient to be treated does not have a neurodegenerative disorder, or does not have a condition in which there is elevated MMP-9 expression or activity. Thus, in some embodiments the disorder to be treated excludes schizophrenia, Fragile-X-Syndrome and/or Alzheimer's disease and other dementia disorders.

Efficacy of treatment of the above conditions using the methods described herein may be monitored in one embodiment by detecting induction of high gamma frequency oscillations, e.g. using EEG methods as described above. Other suitable methods for monitoring efficacy of the treatment may use serum biomarkers, cognitive tests, and/or brain imaging such as magnetic resonance imaging (MRI) or positron emission tomography (PET).

### Promoting cognitive function

In further embodiments, the present invention may be used for promoting or improving cognitive function in a subject. For instance, the methods, devices, systems and compositions described herein may be used to promote or improve learning, attention, memory, language, executive functions, social cognition and/or visual-spatial abilities.

Preferably the methods, devices, systems and compositions described herein are used to promote or improve executive functions, learning, attention or memory. In one embodiment, the present invention is used to promote or improve memory, e.g. working memory, episodic memory, recognition memory, reference memory, visual and/or spatial memory, preferably working memory. In one preferred embodiment, the invention is used to promote or improve memory preservation and retrieval. In another embodiment, the present invention is used to promote or improve learning, preferably reversal learning.

In some embodiments, the present invention may be used to promote cognitive function in a normal subject. By "normal subject" in this context, it is a meant a subject or patient who is not suffering from a clinical disorder, e.g. a neuropsychiatric disorder. Thus a "normal subject" may refer to a healthy subject. The present invention (e.g. the methods, devices, systems and compositions described herein) may be used in non-therapeutic treatment of such subjects. In some embodiments, the present invention may be used to treat subjects experiencing stress or burnout, preferably chronic stress, including e.g. in subjects experiencing non-clinical or sub-clinical signs or symptoms of stress.

### Separate and combination treatments

A skilled person will appreciate that a visual (or auditory) stimulus such as flashing light may have advantages compared to a pharmaceutical composition comprising ketamine, e.g. in terms of the avoidance of side effects associated with this drug. Nevertheless, for other purposes application of light pulses may be considered to be analogous to the administration of a pharmaceutical composition, in the sense that it involves delivery of photons to a subject as part of a therapeutic method.

Accordingly, another aspect described herein provides light pulses (or flashing light or photons, typically within the visible range) for use in medicine. The light pulses typically have a frequency of about 50 to about 70 Hz. The light pulses or photons may be used e.g. to (i) induce synchronized gamma oscillations in at least one brain region of a subject; wherein the synchronized gamma oscillations have a frequency of about 50 to about 70 Hz; (ii) promote removal of the perineuronal net in a brain region of a subject; and/or (iii) promote neuronal plasticity in a brain region of a subject. Thus the light pulses may be used to treat a disease or condition associated with dysfunction in neuronal plasticity. In particular embodiments, the light pulses are used for treating any of the neurological or neuropsychiatric disorders described above, e.g. schizophrenia, bipolar disorder or depression. In a preferred embodiment, methods described herein employing a visual stimulus (e.g. light pulses/a flashing light at a frequency of about 50 to 70 Hz) may be used to treat subjects who cannot tolerate ketamine treatment, e.g. due to side effects associated with this drug.

Methods, systems, devices and compositions described herein may be used alone or in combination in order to promote neuronal plasticity. For instance, light pulses at a frequency of about 50 to about 70 Hz may be applied to a subject in combination with a pharmaceutical composition comprising an active agent, e.g. ketamine. Alternatively light pulses at a frequency of about 50 to about 70 Hz may be applied to a subject in combination with sound (e.g. a click train) at a frequency of about 50 to about 70 Hz, optionally further in combination with a pharmaceutical composition comprising ketamine. In such cases, combined use of multiple treatment modalities may provide synergistic effects in treatment, reduced side effects and/or enable a lower dose of an active agent (e.g. ketamine) to be used.

In some embodiments, light pulses at a frequency of about 50 to about 70 Hz may be applied to a subject in combination with a pharmaceutical composition comprising an active agent used to treat a neuropsychiatric disorder, e.g. schizophrenia, bipolar disorder, post-traumatic stress disorder and/or depression. For instance, the pharmaceutical composition may comprise an antidepressant, an anxiolytic, a psychedelic, an antipsychotic or a neuroleptic drug.

In particular embodiments, the pharmaceutical composition may comprise an active agent selected from one of the following groups or individual agents, or a pharmaceutically acceptable salt thereof:
Antidepressants:
   (i) selective serotonin reuptake inhibitors (SSRIs), e.g. fluoxetine, sertraline, citalopram, escitalopram, fluvoxamine, paroxetine;
   (ii) serotonin-norepinephrine reuptake inhibitors (SNRIs), e.g. venlafaxine, desvenlafaxine, duloxetine, milnacipran, levomilnacipran;
   (iii) serotonin antagonist and reuptake inhibitors (SARIs), e.g. trazodone, nefazodone;
   (iv) serotonin modulator and stimulator (SMSs), e.g. vortioxetine, vilazodone;
   (v) norepinephrine reuptake inhibitors (NRIs), e.g. reboxetine, atomoxetine, teniloxazine, viloxazine;
   (vi) norepinephrine-dopamine reuptake inhibitors (NDRIs), e.g. bupropion;
   (vii) tricyclic antidepressants (TCAs), e.g. amitriptyline, amoxapine, desipramine, doxepine, imipramine, nortriptyline, protriptyline, trimipramine, clomipramine, maprotiline;
   (viii) tetracyclic antidepressants (TeCAs), e.g. mirtazapine, amoxapine, maprotiline, mianserin, setiptiline;
   (ix) monoamine oxidase inhibitors (MAOIs), e.g. isocarboxazid, phenelzine, selegiline, tranylcypromine, rasagiline;
   (x) others, e.g. agomelatine, ketamine, esketamine, lithium, buspirone, modafinil, lamotrigine;
Antipsychotics/neuroleptics:
   (xi) typical antipsychotics, e.g. haloperidol, loxapine, thioridazone, molindone, thiothixene, fluphenazine, mesoridazine, trifluoperazine, perphenazine, chlorprothixene, pimozide, prochlorperazine, acetophenazine, triflupromazine;
   (xii) atypical antipsychotics, e.g. aripiprazole, brexpiprazole, olanzapine, quetiapine, risperidone, lurasidone, amisulpride, clozapine, ziprasidone, cariprazine;
Anxiolytics:
   (xiii) barbiturates, e.g. phenobarbital;
   (xiv) benzodiazepines, e.g. alprazolam, bromazepam, chlordiazepoxide, clonazepam, clorazepate, diazepam, flurazepam, lorazepam, oxazepam, temazepam, triazolam, bromdihydrochlorphenylbenzodiazepine;
   (xv) carbamates, e.g. meprobamate, carisoprodol, tybamate and lorbamate;
   (xvi) antihistamines, e.g. hydroxyzine, chlorpheniramine and diphenhydramine;
   (xvii) opioids, e.g. hydrocodone, fentanyl, buprenorphine;
Psychedelics;
   (xviii) e.g. lysergic acid diethylamide (LSD), psilocybin, cannabis, ayahuasca, ololiuqui, 3,4-methylenedioxymethamphetamine (MDMA), mescaline, ibogaine, salvinorin A, 2,5-dimethoxy-4-methylamphetamine (DOM), 2,5-dimethoxy-4-bromophenethylamine (2C-B), 25I-NBOMe (2-(4-iodo-2,5-dimethoxyphenyl)-N-[(2-methoxyphenyl)methyl]-ethanamine), and extracts/derivatives thereof.

Pharmaceutical compositions comprising the above active agents are known in the art. Suitable pharmaceutical compositions may further comprise a pharmaceutically acceptable carrier, diluent, excipient, or buffer. In some embodiments, the pharmaceutically acceptable carrier, diluent, excipient, or buffer may be suitable for use in a human.

Pharmaceutically acceptable salts of the active agents mentioned above may be used, for example, mineral acid salts such as hydrochlorides, hydrobromides, phosphates, sulphates, and the like; and the salts of organic acids such as acetates, propionates, malonates, benzoates, and the like. Additionally, auxiliary substances, such as wetting or emulsifying agents, pH buffering substances, and the like, may be present in such vehicles. A wide variety of pharmaceutically acceptable excipients are known in the art and need not be discussed in detail herein. Pharmaceutically acceptable excipients have been amply described in a variety of publications, including, for example, A. Gennaro (2000) "Remington: The Science and Practice of Pharmacy," 20th edition, Lippincott, Williams, & Wilkins; Pharmaceutical Dosage Forms and Drug Delivery Systems (1999) H. C. Ansel et al., eds., 7(th) ed., Lippincott, Williams, & Wilkins; and Handbook of Pharmaceutical Excipients (2000) A. H. Kibbe et al., eds., 3 rd ed. Amer. Pharmaceutical Assoc. The pharmaceutical compositions described herein may be e.g. solid or liquid dosage forms for oral administration including capsules, tablets, pills, powders, solutions, and granules. Suitable dosages of the defined active agents are also known in the art, or may be selected by a skilled person based on e.g. clinical criteria.

In particular embodiments, the methods described herein may be used to sustain a response to an active agent as described above, e.g. an antidepressant, an anxiolytic, a psychedelic, an antipsychotic or a neuroleptic drug. For instance, light pulses at a frequency of about 50 to about 70 Hz may be applied to a subject at intervals between treatments with the active agent. For instance, a subject may be treated with an active agent such as ketamine or a psychedelic drug under controlled or clinically-supervised conditions, e.g. in a hospital or other clinical setting. In order to sustain or promote the response to such a treatment (e.g. between hospital visits, which may be 1 month or more apart), the subject may be treated at home or under non-clinically supervised conditions (e.g. daily or weekly) with light pulses at a frequency of about 50 to about 70 Hz. By "clinically supervised" it is meant conditions where a medically qualified professional (e.g. nurse or doctor) is required to be present.

In some embodiments, the methods described herein may be combined with exposure to specific environments or stimuli, such as performing mental exercises (e.g. games), e.g. in order to further improve cognitive abilities or mindset during the period of treatment. Without being bound by theory, in some embodiments the treatments described herein may result in removal of the PNN, which may be followed by a period in which the PNN is reformed in an improved configuration. Therefore exposure to specific environments or stimuli, such as performing mental exercises during the period in which PNNs are being rebuilt, e.g. within 1 day to 14 days after the treatment described herein, may result in synergistic improvements in treatment. Examples of specific environments or stimuli include mental or cognitive challenges and exercises, monocular deprivation, fear extinction training, psychosocial therapy, learning and relearning, psychotherapy, behavioural therapy, trauma therapy, and exposure and response prevention (ERP) therapy.

In some such embodiments, a stimulus (e.g. light at 60 Hz) is applied to the subject during exposure to such specific environments or stimuli. As demonstrated in Example 2 below, the systems, devices and methods of the present invention may advantageously improve cognitive function and alleviate neuropsychiatric disorders when the stimulus exposure is concurrent with a mental challenge, such as learning or re-learning a task. Thus in some embodiments, a stimulus may be applied to the subject during (i.e. simultaneously with) mental or cognitive challenges and exercises, monocular deprivation, fear extinction training, psychosocial therapy, learning and relearning, psychotherapy, behavioural therapy, trauma therapy, and exposure and response prevention (ERP) therapy.

### Subjects

The term "subject" or "patient" as used herein typically denotes humans, but may also encompass reference to non-human animals, preferably warm-blooded animals, more preferably viviparous animals, even more preferably mammals, such as, *e.g.,* non-human primates, rodents, canines, felines, equines, ovines, porcines, and the like.

### Definitions

Unless otherwise specified, all terms used in disclosing the invention, including technical and scientific terms, have the meaning as commonly understood by one of ordinary skill in the art to which this invention belongs. By means of further guidance, term definitions may be included to better appreciate the teaching of the present invention.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The term also encompasses "consisting of" and "consisting essentially of'.

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of and from the specified value, in particular variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The present invention will now be described by way of example only with reference to the following non-limiting embodiments.

### EXAMPLES

### Methods

### Animals:

Adult animals (8-12 weeks) of both sexes were used. C57BL/6J (#000664) animals were purchased from The Jackson Laboratories. All animals were housed in the IST Austria Preclinical Facility, with 12 hours light-dark cycle, food and water provided *ad libitum.* All animal procedures are approved by the "Bundesministerium für Wissenschaft, Forschung und Wirtschaft (bmwfw) Tierversuchsgesetz 2012, BGBI. I Nr. 114/2012 (TVG 2012) under the number GZ BMWF-66.018/0001-II/3b/2014.

### Drug application:

C57BL/6J mice were intraperitoneal (*i.p.*) injected in the morning. Deep anaesthesia was confirmed based on the following parameters:
1. Absence of the toe pinch reflex around 10 minutes after induction.
2. Decrease in the respiratory frequency.
3. No responses to external stimuli.
4. Flaccid paralysis.
5. Absence of whiskers movement.

To prevent corneal dehydration, eye ointment (Oleo Vital) was applied. During the procedure and recovery phase, animals were kept at 37°C.

*Ketamine Xylazine Acepromazine (KXA):* ketamine (100 mg/Kg, MSD Animal Health), xylazine (10 mg/Kg, AniMedica) and acepromazine (3 mg/Kg, VANA GmbH) was solubilised in physiological saline solution containing 0.9% (w/v) of NaCl (Fresenius Kabi Austria). Solution was always freshly prepared to avoid pH fluctuations.

*Xylazine Acepromazine (XA)*: same as KXA but omitting ketamine.

*Control:* The same volume of physiological saline solution was injected as for KXA.

*Repeated KXA-anaesthesia:* Animals were exposed either 1x, 2x, 3x, or 6x with KXA-anaesthesia with 3-4 days difference between the treatments (Hohlbaum, K. *et al* (*supra*)).

*Clopidogrel:* Clopidogrel (Tocris 2490) was dissolved in dimethyl sulfoxide (DMSO, Sigma, D8418) and diluted 1:4 in phosphate buffer saline (PBS) before the injection. 50 mg/Kg Clopidogrel were *i.p.* injected 5 minutes before KXA-anaesthesia, which reaches peak plasma levels within 1 min following intravenous administration.

### Light flicker stimulation:

The stimulation box consisted of a black plastic box with a lid (OBI Tauro 62 Liters, 60x40x32cm). A strip of light emitting diodes (LEDs, RS Components, Part. N. LSWW61210MIP20) was attached to the side walls of the box. The flicker frequency was set at 8, 40 and 60 Hz with a square wave current pattern generated using an Arduino system (Arduino UNO SMD, Figure 1a). The frequency of flickering was verified using a photoresistor (5 mm GL5516 LDR Photo Resistance), controlled by an Arduino UNO, positioned 1 cm away from the LED strip (see Figure 1b). Images for circuit diagram were generated with fritzing app (version 0.9.3b). In the centre of the stimulation box, 3.9*10¹⁸ photons/cm²/s were generated.

Mice were transported from the holding room of the animal facility to the laboratory. Before the cage was put in the centre of the stimulation box, 8 cm away from the LEDs, all nesting materials and tools were removed. Animals were always exposed to the light stimulation protocols (2 hours for 5 consecutive days) in the morning. During the stimulation, mice were allowed to move freely inside their cage with access to food and water *ad libitum.* After the stimulation, the animals received nesting material and were transferred back to the facility.

Control animals underwent the same transport and lab environment but were only exposed to normal room light intensity.

### Tissue Preparation:

All tissues were dissected 4h after the last post-drug treatment passing the KXA half-life of 2-3 h (Zhang K. et al (2018) Sci. Rep. 8(1):4007). For histological analysis, animals were shortly anesthetized with isoflurane (Zoetis) and secured to the perfusion plate. The chest was open to expose the hearth. The left ventricle was cannulated, and the *inferior vena cava* cut. The animals were initially perfused with 20 mL of phosphate-buffered saline (PBS) with heparin (100 mg/L, Sigma H0878), followed by 20 mL of 4% (w/v) paraformaldehyde (PFA, Sigma P6148) in PBS using a peristaltic pump (Behr PLP 380, speed: 25 rpm). The animals were decapitated, the retina and brain explanted, and post-fixed in 4% (w/v) PFA/PBS for 30 minutes and overnight (16h), respectively. Then, the tissues were washed in PBS and stored at 4°C with 0.025% (w/v) sodium azide (VWR 786-299). For cryoprotection, the tissue was transferred in 30% (w/v) sucrose (Sigma 84097) in PBS and incubated overnight at 4°C. To increase antibody permeability, the brain slices were frozen over dry-ice and thawed at room temperature for three cycles. Then, the brain was sliced in 100 µm coronal slices on a vibratome (Leica VT 1200S), if not otherwise indicated.

### Immunohistochemistry:

The tissue was incubated with blocking solution containing 1% (w/v) bovine serum albumin (Sigma A9418), 5% (v/v) Triton X-100 (Sigma T8787), 0.5% (w/v) sodium azide (VWR 786-299), and 10% (v/v) serum (either goat, Millipore S26, or donkey, Millipore S30) for 1 hour at room temperature on a shaker. Afterwards, the samples were immunostained with primary antibodies diluted in antibody solution containing 1% (w/v) bovine serum albumin, 5% (v/v) triton X-100, 0.5% (v/v) sodium azide, 3% (v/v) goat or donkey serum (see Table 1), and incubated for 48 hours on a shaker at room temperature.

**Table 1:**

| Antibody | Dilution | Company |
|---|---|---|
| Rabbit α-Caspase3 | 1:400 | Cell Signaling (9661S) |
| Rat α-CD68 | 1:250 | AbD Serotec (MCA1957) |
| Rabbit α-GFAP | 1:500 | Dako (Z0334) |
| Goat α-Iba1 | 1:250 | Abcam (ab5076) |
| Rabbit α-Iba1 | 1:750 | GeneTex (GTX100042) |
| Guinea pig α-parvalbumin | 1:500 | SYSY (195004) |
| Chicken α-parvalbumin | 1:1000 | Novus Biologicals (NBP2-50036) |
| *Wisteria floribunda* lectin-fluorescein-labelled | 1:200 | Szabo-Scandic (VECFL-1351) |
| *Wisteria floribunda* lectin - biotinylated | 1:200 | Szabo-Scandic (VECB-1355) |

The slices were then washed three times with PBS and incubated light-protected with the secondary antibodies diluted in antibody solution for 2 hours at room temperature on a shaker. The secondary antibodies raised in goat or donkey were purchased from Thermo Fisher Scientific (Alexa Fluor 488, Alexa Fluor 568, Alexa Fluor 647, 1:2000). The slices were washed three times with PBS. The nuclei were labelled with Hoechst 33342 (Thermo Fisher Scientific, H3570, 1:5000) diluted in PBS for 15 minutes. The slices were mounted on microscope glass slides (Assistant, 42406020) with coverslips (Menzel-Glaser #0) using an antifade solution (10% (v/v) mowiol (Sigma, 81381), 26% (v/v) glycerol (Sigma, G7757), 0.2M tris buffer pH 8, 2.5% (w/v) Dabco (Sigma, D27802)).

### Confocal microscopy:

Images were acquired with a Zeiss LSM880 upright Airyscan or with a Zeiss LSM880 inverted fast Airyscan using a Plan-Apochromat 40X oil immersion objective N.A. 1.4. Images were acquired as 2x2 tile scan z-stacks with a resolution of 0.208 x 0.208 x 0.371 µm.

### Image analysis:

Confocal images were loaded in Fiji 1.52e (http://imagej.net/Fiji). To remove the background the rolling ball radius was set to 35 pixels, and images were filtered using a median 3D-filter with x, y, z radius set at 3. Image stacks were exported as .tif files, converted in .ims files using Imaris converter, and imported in Imaris 8.4.2.v (Bitplane Imaris).

*Perineuronal net density per mm³.* In each image, neurons surrounded by perineuronal nets were counted using the spot detection function of Imaris (Oxford Instruments). The total cell count was normalised to the entire image volume and data are represented as number of cells surrounded by perineuronal nets per mm³.

*PNN volume within microglial CD68.* Surface renderings were generated on microglia, CD68, and WFA/PNN z-stacks using the surface rendering module of Imaris 8.4.2. Surfaces were generated with the surface detail set to 0.2 µm. In order to obtain the CD68 surface within microglia or the surface of WFA within the microglial CD68, the surface-surface coloc plugin was used. This analysis was performed on the entire image. The total percentage of perineuronal net volume within this microglial CD68 volume was calculated per image.

*Cell density per mm³.* In each image, either parvalbumin⁺-neurons or Iba1⁺-microglia were counted using the spot detection function of Imaris. The total cell count was normalised to the entire image volume and data are represented as number of cells per mm³.

### Statistical analysis:

All statistics were performed with R (version 3.4.4), if not otherwise indicated. Models were generated by changing the default contrast for unordered variables (e.g. experimental condition) to "contr.sum". This allows to apply type III anova on the model to evaluate the overall contribution of unordered effects on the response variable. Reported P-values of post-hoc tests (performed via the "multcomp" package) were corrected for multiple testing according to the default *single-step method.* If not otherwise indicated, all possible pairwise comparisons were performed. If not, others indicated: Linear regression was performed with the Lme4 package (version 1.1-17). Error bars represent standard error of the mean, calculated with *mean_se()* as part of *hmisc* package in *ggplot2*). Analysis results were exported from Imaris into an excel file, which was loaded into R via the *xlsx* package (version 0.6.1). Plots were generated with ggplot2 (version 3.0.0). * p = 0.05, ** p = 0.01, *** p = 0.001.

*Combined controls:* To compare experimental groups subjected to different injection paradigms, controls that received 1x, 3x, or 6x saline injections were first compared and a mean value obtained from the means of these conditions (combined controls), which served as control for further statistical testing.

*Perineuronal net density per mm³:* Each data point represents the count of neurons surrounded by PNN in one image per animal (biological replicates). For all following analyses, cell densities were calculated as a percentage with the respective control set to 100%. Linear regression was used to predict cell density per mm³ (as absolute values or as percentages compared to the respective control) by experimental condition in cases where more than two levels were involved. Then, a two-sided T-test was then used to investigate differences between experimental conditions in cases where only two levels were involved. P-values were adjusted with the "p.adjust" function and the method set to "BH".

*Gender-differences:* To compare removal and recovery of perineuronal nets between the sexes, differences between male and female were tested within each experimental group via linear regression and selected posthoc contrasts for the desired comparisons.

*PNN volume within microglial CD68:* Each data point represents several microglia within one image per animal (biological replicates). Linear regression was used to predict the square root-transformed percentage of PNN volume within microglial CD68 volume by experimental condition. For CD volume within microglia, linear regression was used to predict the percentage of CD68 volume within microglia volume by experimental condition.

*Cell density per mm³:* Each data point represents neurons stained for parvalbumin or microglia stained for Iba1 within one image per animal (biological replicates). A two-sided T-test was used to investigate differences between experimental conditions in cases where only two levels were involved. P-values were adjusted with the "p.adjust" function and the method set to "BH".

### Results

### Repeated ketamine exposure results in PNN loss

To establish the consequences of ketamine-exposure on PNN, adult C57BL/6J animals were subjected to single or repeated ketamine-anaesthesia with 3 days-intermediate intervals, and collected the brains 4h after either 1x, 2x, 3x, or 6x injections (Figure 2a). The anaesthetic dosage focused on was to achieve the maximum pharmacological effect and the same phenotypically readout across animals of both sexes. Since ketamine at anaesthetic dosage induces muscle rigidity, it was combined with xylazine together with the phenothiazine tranquiliser acepromazine (KXA) (Arras, M. et al (2001) Comp. Med. 50(2):160-166). The PNN was stained with *Wisteria floribunda* agglutinin (WFA), which shows widespread distribution in the brain (Figure 2b), and preferentially surrounds cortical parvalbumin-positive interneurons (Figure 2c). Male and female showed the same baseline density of PNN-covered cells and neither repeated saline injection nor 1x KXA changed this number (Figure 2d-e). Strikingly, PNN loss started to be observed after 2x KXA, which was almost gone after 3x KXA, and remained gone after 6x KXA (Figures 2f-i). To exclude that PNN loss was mediated by xylazine-acepromazine, animals were analysed which received three repeated xylazine-acepromazine-only injections (3x XA). Neither the PNN distribution nor the number of PNN-covered cells were altered upon 3xXA (Figures 2j-k) suggesting that the anaesthetic dosage of ketamine was the main driving component of PNN loss.

### PNN loss temporarily re-opens brain plasticity window

PNN maturation closes the critical period of brain plasticity, and enzymatic digestion of the extracellular matrix with chondroitinase-ABC has been shown to restore plasticity (Berardi, N. et al (2004) Neuron 44(6):905-908; Orlando, C. et al (2012) J. Neurosci. 32(50):18009-17; Levy, A. D. et al (2014) Front. Neuroanat. 8:116; De Vivo, L. (2013) et al. Nat. Commun. 4:1484; Pizzorusso, T. et al (2002) Science 298(5596):1248-51; Gogolla, N. et al (2009) Science 325(5945):1258-61; Happel, M. F. K. et al (2014) Proc. Natl. Acad. Sci. 111(7):2800-5). The binocular region of the primary visual cortex (V1) establishes the contralateral eye ocular dominance (OD) during development. Similar to S1, this study found that 3x KXA results in PNN loss in V1 (Figures 3a-b), which was independent of XA (Figure 3c) and the strain background (Figure 3d). Since the PNN was still significantly reduced 3 days after recovery of 3x KXA, repeated ketamine-induced PNN loss may temporarily re-open a plasticity window. For instance, PNN loss could reactivate ocular dominance (OD) plasticity in adult animals.

### Microglia are responsible for PNN loss - Microglia selectively remove the PNN but not parvalbumin neurons.

To identify the underlying mechanism of PNN loss, it was necessary first to exclude that repeated ketamine exposure induced apoptosis and altered the density of parvalbumin-positive neurons in the cortex. After also ruling out astrogliosis (Figure 4a), microglia were suspected as the primary source because they are in the unique position to alter brain plasticity (Kettenmann, H. et al (2011) Physiol. Rev. 91(2):461-553). Besides sensing neuronal activity, microglia can strengthen or eliminate neuronal connections during circuit formation and degeneration (Schafer, D. P. et al (2012) Neuron 74:691-705; Paolicelli, R. C. et al (2011)Science 333:1456-1458; Kettenmann, H. et al (2013) Neuron 77(1):10-18; Tremblay, M. Ě. *et al* (2010) *PLoS Biol.* 8(11):e1000527). The density of Iba1-positive microglia was not altered upon repeated KXA. However, the endosomal lysosomal marker CD68 within microglia significantly increased indicating microglia engaged in phagocytic activity (Kettenmann, H. *et al (supra);* Bauer, J. et al (1994) J. Neurosci. Res. 38(4):365-75; Alessandrini, F. et al (2017) Seminars in Oncology 44(4):239-253) (Figure 4b-c). It was found that, with 2x KXA, microglia were in close proximity to PNN-covered cells and contained PNN fragments within CD68 vesicles at multiple locations in the field-of-view (Figure 5), which was not observed in saline injected animals (Figures 6 and 7). Overall, the PNN volume within microglial CD68 was the highest at 3x KXA, confirming that microglia are actively involved in removing PNN without affecting the parvalbumin-positive population.

### Inhibiting microglial chemotactic response prevents PNN loss upon repeated ketamine exposure

Next, a chemotactic microglia response was elicited via the purinergic receptor P2Y12, which is expressed on the microglial surface (Sipe, G. O. et al (2016) Nat. Commun. 7:10905; Haynes, S. E. et al (2006) Nat. Neurosci. 9(12):1512-9). To elucidate whether P2Y12 is involved, the P2Y12 selective-drug, clopidogrel, was injected 5 minutes before the KXA-injection (Figure 8a) (Savi, P. et al (2001) Biochem. Biophys. Res. Commun. 283:379-383). It was found that *a priori* clopidogrel injection prevented ketamine-mediated loss of PNN-covered cells (Figure 8b-c), and microglia showed less interaction with the PNN structure. These results support the theory that microglia are the main mediator of PNN loss.

### Ketamine-mediated 60 Hz frequency stimulates microglia to PNN removal

Next, how microglia are stimulated to remove PNN was investigated together with how this is connected to ketamine: neuronal activity was thought to be a trigger. Microglia have been known to respond to changes in neuronal activity and, when P2Y12 was inhibited, microglia did not remove the net. When the brain was investigated after 3x KXA, massive upregulation of the early gene marker cFos was found.

Previous studies have shown that ketamine dynamically alters the theta and gamma frequency bands in rats and cats and does not induce bursts typically induced during epileptic seizures (Ahnaou, A. et al (2017) Transl. Psychiatry 7(9):e1237; Castro-Zaballa, S. et al (2019) Front. Psychiatry 2:766). To test whether one of these frequencies triggers microglia to remove PNN in the V1, a light flickering stimulus was applied at either 8 or 60 Hz for 2h, which is the half-life of ketamine, for 5 days (Figure 9a). 40 Hz stimulation was used as a control which has been previously shown to be sufficient to trigger microglia to remove amyloid plaques in Alzheimer's mouse models (Iaccarino, H. F. et al (2016) Nature 540(7632):230-235; Adaikkan, C. et al (2019) Neuron 102(5):929-943.e8).

It was found that the density of PNN-covered cells reduced by 30% upon 60 Hz stimulation, whereas 8 Hz, and 40 Hz, did not show an effect (Figure 9b). A 60 Hz flickering light stimulus was therefore capable of reducing the density of PNN-covered cells to a similar degree to 2x KXA stimulation (compare e.g. Figure 2I). A 60 Hz flickering light stimulus also produced a similar increase of cFos expression in the brain as 3x KXA stimulation. However the density of microglia did not change following 60 Hz stimulation, it contrast to the effect of 40 Hz stimulation reported by Iaccarino, H. F. *et al* (2016), *ibid.* Following the 60 Hz stimulus, microglia were found to be in close proximity to the PNN and to digest it.

The above results show that repeated ketamine exposure results in an increase in 60 Hz frequency gamma oscillations in the brain, which triggers microglia to eliminate the PNN. A similar effect can be induced by a 60 Hz flickering light stimulus.

### Microglia show layer-specific MMP-9 upregulation.

Previous studies have shown that matrix metalloproteinases play a critical role in juvenile and adult OD-plasticity. One candidate is matrix metalloproteinase-9 (MMP-9), which is released in a neuronal activity-dependent manner, and has been shown to degrade Pvalb⁺-neuron PNN in the context of glioma. The source of MMP-9 could be either Pvalb⁺-neuron themselves or glia cells, therefore, we performed immunostaining after 3x KXA for Pvalb, S100β and Iba1 and analyzed the MMP-9 volume within Pvalb⁺-neurons, astrocytes, and microglia, respectively. Pvalb⁺-neurons showed a significant MMP-9 increase upon 3x KXA, whereas astrocytes lacked this effect. Remarkably, microglia located in cortical L3-5 significantly elevated their MMP-9 levels, whereas microglia in L1 did not show this effect. This layer-selective upregulation corresponds also to the location of PNN-coated Pvalb⁺-neuron in S1. To further support a closer interaction between microglia and Pvalb⁺-neurons, we determined the distance between microglia and Pvalb⁺-neuron, which was reduced upon 3x KXA.

In parallel, we also tested an alternative, activity-dependent matrix metalloproteinase-14 (MMP-14), which is known to be upregulated in microglia in distinct neuroinflammatory conditions. However, we did not observe any MMP-14 upregulation in any of the three cell classes suggesting a selective ECM endopeptidase response.

Overall, an activity-dependent relationship exists between Pvalb⁺-neurons and microglia that results in a layer-specific upregulation of MMP-9 in microglia upon ketamine exposure and upon flickering light exposure at 60Hz, indicating a potential role of microglia in PNN disassembly.

### Impact of 60Hz flickering light in a learning and reversal-learning mouse model

C57BL/6J wild-type adult females were used for the learning and reversal-learning experiments with the Intellicage system. One week before the beginning of the experiments, the animals were implanted with a subcutaneous transponder (under light Isoflurane anesthesia) for tracking their movements. The intellicage system consists of a cage equipped with 8 water battles, 2 per corner. The access to the water is modulated by a gate with integrated nose poke and presence sensors. The advantage of the intellicage system is that allows automatic and unbiased real time tracking of the animals behavior without any interaction between the mice and the experimentalists.

The experimental strategy is shown in Figure 12. The mice were randomly divided in 2 experimental groups, one receiving constant light and one 60 Hz light enrichment. The behavioural paradigm started with a habituation phase in which the mice have to nose poke and wait 10 seconds in order to have access to the water. In the next phase, called place learning, the access to only one water bottles was randomly assigned to each animal. In the following reversal learning phase, the access to water was switched to the opposite water bottle. At the end of the experiments the animals entered in the final stage, in which they had unlimited access to all the corners. A part of the habituation phase, in which 100 correct trials were required, in all the other stages the animals had to perform 500 correct trial to go to the next phase of the experiments.

Figure 13 shows the time taken for 60 Hz light-treated ("Flick") and constant light-treated ("Light") mice to complete 30 successful trials in the place learning and reverse learning phases. The time taken by 60 Hz light-treated mice (mean of around 31 hours for place learning and 23 hours for reversal learning) was much lower than for constant light-treated mice (mean of around 45 hours for both place and reversal learning.

Figure 14 shows the percentage of side-error mistakes made by 60 Hz light-treated ("Flick") and constant light-treated ("Light") mice in the place learning and reverse learning phases. The percentage of side-error mistakes made by 60 Hz light-treated mice (mean of around 25% for place learning and 15% for reversal learning) was much lower than for constant light-treated mice (mean of around 34% for place learning and 25% for reversal learning).

Our behavioural results clearly showed that the 60 Hz light-exposed animals completed each phase significantly faster, and with less errors, compared to the light-control mice. This suggests that 60 Hz light enrichment made the brain more plastic, accelerating consolidation and formation of new memories, leading to increased learning and reversal-learning performance in mice.

### Discussion

The results presented herein show that ketamine triggers microglia to remove the PNN, which results in reopening of the plasticity window in the primary visual cortex. The microglia response is partially induced by the 60 Hz frequency oscillation that naturally occurs during ketamine anaesthesia and is enhanced upon repeated stimulation. The same response can be induced by a 60 Hz flickering light stimulus. The response to ketamine and the flickering light stimulus is shown here to be associated with a specific increase in expression of matrix metalloproteinase 9 (MMP-9). MMP-9 activity plays an essential role in synaptic plasticity and long-term potentiation, and therefore in learning and memory. MMP-9 is known to be upregulated in response to administration of monoamine oxidase antidepressants, and this upregulation is believed to contribute to the long-term positive effects of antidepressant medication such as SSRIs.

The present invention therefore includes in one aspect a method of upregulating MMP-9 activity in the brain by exposure to a flickering light stimulus in the range 50-70 Hz, preferably about 60 Hz. Since this non-invasive light treatment can achieve upregulation of MMP-9 it can be used for treatment of any condition that is conventionally treated with antidepressants, such as all forms of depression, anxiety and obsessive-compulsive disorders.

In our model of learning/reversal-learning, mice showed a significant improvement in speed and accuracy of learning/reversal-learning when subjected to flickering light at a frequency of about 60Hz. This remarkable finding has great clinical significance. It shows that this simple intervention can profoundly affect the ability of the brain to forge new neuronal connections and consolidate newly-acquired memories. If these new connections can be directed in a positive manner there is the potential to be able to influence or even reverse existing patterns of thoughts or behavior, thereby offering new therapeutic options for disorders such as phobias, PTSD, eating disorders, schizophrenia and OCD.

This study reveals a novel way of how microglia shape the neuronal environment, in which they almost completely eradicate the PNN. As a specialised extracellular matrix compartment, the PNN forms around selected neurons during critical periods in multiple brain regions (Celio, M. R. & Blumcke, I. *(supra);* De Vivo, L. *et al (supra);* Pizzorusso, T. *et al (supra);* Gogolla, N. *et al (supra);* Hensch, T. K. (2005) Nature Reviews Neuroscience 6(11):877-88; Frischknecht, R. & Gundelfinger, E. D. (2012) Adv. Exp. Med. Biol. 970:153-71; Saghatelyan, A. K. et al (2001) Mol. Cell. Neurosci. 17(1):226-40; Härtig, W. et al (1999) Brain Res. 842(1):15-29; Alberini, C. M. & Travaglia, A. (2017) J. Neurosci. 32:9429-9437; Lau, L. W. et al (2013) Nature Reviews Neuroscience 14(10):722-9). Consequently, it sterically restricts synapse formation and receptor mobility, which suppresses neuronal plasticity in adulthood (Pizzorusso, T. *et al (supra);* Gogolla, N. *et al (supra);* Hensch, T. K. *(supra);* Frischknecht, R. & Gundelfinger, E. D. *(supra);* Carstens, K. E. et al (2016) J. Neurosci. 36(23):6312-20).

Enzymatic digestion of the extracellular matrix with chondroitinase ABC injection into the brain resulted in enhanced cognitive flexibility in the visual cortex (Pizzorusso, T. *et al* (*supra*)), amygdala (Gogolla, N. *et al (supra))* and auditory cortex, among others (Happel, M. F. K. *et al (supra);* Banerjee, S. B. et al (2017) Neuron 95(1):169-179.e3). The experiments presented herein have shown that the removal of this critical barrier temporary re-opens plasticity and could restore ocular dominance to the juvenile level.

This information provides a new clinical frame for the use of ketamine in human therapy. For example, patients of complex regional pain syndrome are alleviated from neuropathic pain after either an induced ketamine coma for five days (Kiefer, R. T. *et al* (*supra*); Becerra, L. *et al* (*supra*)) or upon repeated exposure to subanaesthetic dosage (Goldberg, M. E. *et al* (*supra*))*.* The latter paradigm has known benefits for patients suffering from depression (Krystal, J. H. *et al* (*supra*); Berman, R. M. *et al* (*supra*)). A recent study in mice found that ketamine treatment restored stress-induced spine loss and depression-related behaviour (Moda-Sava, R. et al (2019) Science 364(6436):pii:eaat8078). That ketamine results in increased dendritic spine formation and motility, as well as reverting existing spines into an immature phenotype has been shown in multiple studies (Orlando, C. *et al* (*supra*); Levy, A. D. *et al* (*supra*); De Vivo, L. *et al* (*supra*)). The studies described herein enable the use of ketamine in clinical situations where it is desirable to promote neuronal plasticity, particularly via removal of the PNN.

The promising impact of ketamine in a disease environment has to be put in contrast with repeated ketamine exposure in healthy individual. In humans, repeated long-term ketamine exposure for recreational purposes leads to memory impairments and schizophrenic-like symptoms (Morgan, C. J. A. *et al* (*supra*); Adler, C. M. *et al* (*supra*)), and mouse behaviour studies with repeated ketamine-xylazine treatment found increased anxiety in female mice (Hohlbaum, K. *et al* (*supra*); Strong, C. E. & Kabbaj, M. (*supra*)).

The present study was carried out in both sexes due to known gender-specific effects and it was that the action of repeated ketamine exposure was accelerated in females. However, the recovery rate was similar.

In summary, the present experiments demonstrate that repeated ketamine administration at an anaesthetic dosage resulted in loss of perineuronal net (PNN), a specialised extracellular matrix which restricts neuronal plasticity. This loss could therefore promote neuronal plasticity (e.g. ocular dominance plasticity) in the primary visual cortex. Microglia were identified as the critical mediator because blocking their purinergic receptor P2Y12 prior ketamine administration prevented PNN loss. Power spectrum analysis of repeated ketamine exposure pointed to increased high-gamma oscillation frequency. When the frequency was recapitulated with light-flickering, microglia were found to remove PNN. This study provides novel insights into how ketamine may act on brain circuits, as well as outlining a new strategy of how microglia alter adult brain plasticity without physically removing synapses. This provides new clinical uses of ketamine and a flickering light stimulus at about 60 Hz for promoting brain plasticity.

### Example 2 - Effect of flickering light stimulation on working memory

### Background

C57BL/6J wild-type adult males were used for hole-board discrimination learning tasks (see Kuc et al. (2006); Hole-board discrimination learning in mice to assess spatial working- and reference-memory performance. Genes, Brain and Behavior, 5(4):355-363). The hole-board apparatus consists of an open-field chamber with a 16-hole floor insert. Reward pellets (also referred to as 'bait') are placed in four holes, and, across trials, the mice learn the location of these pellets. Normally, mice show learning of the pellet locations within 4 days when completing six trials per day. Various data can be collected, such as data relating to working-memory errors (holes already visited), reference-memory errors (entries to non-baited holes), and errors of omission (missing a baited hole). Reversal training, using new reward pellet locations, can indicate the ability of the animals to actively suppress reward-related responses and to disengage from ongoing behavior.

### Method

### Animals

C57BL/6J (#000664) animals were purchased from Jackson Laboratories. All animals were housed in the IST Austria Preclinical Facility, with 12 hours light-dark cycle, food and water provided *ad libitum.*

### Hole-board paradigm

Seven C57BL/6J adult male mice were used for this experiment. Mice were weighed daily. A timeline of the hole-board paradigm is shown in Figure 10.

### Habituation

Three days before testing, mice were handled daily for 10 minutes per mouse to allow them to become accustomed to the experimenter. Food was also restricted to 10 reward (bait) pellets (#F0071, Bio-serv) per mouse for days 1-2, and 10 reward pellets per mouse + 10% of body weight in normal food for day 3.

### Hole-board training

### Phase 2 - Hole-board training (location 1)

The mice then underwent three days of hole-board training trials with reward pellets placed in holes 5, 7, 10 and 16 (referred to herein as `location 1'). Six trials of three minutes each were completed per day. Each day, two hours after the final trial, the mice received 10% of their body weight in normal food.

The next day, the mice underwent a trial without bait pellets (referred to herein as a `probe trial' - probe trial #1.1). Following the probe trial, the mice received twenty-four reward pellets plus 10% of their body weight in normal food. Mice were given a three-day rest period where they received ten reward pellets plus 15% of their bodyweight in normal food for the first two days. On the third rest day the mice received twenty-six reward pellets plus 15% of their bodyweight in normal food.

Mice then underwent a single hole-board training session with reward pellets in location 1, before undergoing an unbaited probe trial (probe trial #1.2) that afternoon. After the probe trial, mice were given twenty reward pellets plus 10% of their bodyweight in normal food. An additional training session for location 1, and an additional unbaited probe trial (probe trial #1.3) was repeated the next day, and mice given twenty reward pellets plus 10% of their bodyweight in normal food. For the following three days, the mice were allowed to rest as detailed above.

### Phase 3 - Reversal training (location 2)

Following the rest period, mice underwent reversal training. Reversal training is conducted as detailed for the hole-board training above (phase 2) but with reward pellets placed in different holes (location 2). The mice underwent four days of hole-board training trials for location 2, with six trials of three minutes each per day. Each day, two hours after the final trial, the mice received 10% of their body weight in normal food. On the fifth day, the mice underwent a single additional training trial using bait in location 2 before undergoing an unbaited probe trial (probe trial #2.1). Following the trial, mice were given twenty reward pellets plus 10% of their body weight in normal food.

### Hole-board training with constant light

### Constant-light exposure

During this hole-board training stage, the mice received exposure to constant light for two hours per day using the Eurobox system, including rest days and experiment days. Feeding was carried out in a separate cage to ensure that the mice received the correct food dosage.

### Phase 4 - hole-board training with constant light (location 3)

Mice were allowed to rest for two days following the last probe trial and received daily light stimulation as detailed above. On the first day, mice received ten reward pellets plus 15% of their bodyweight in normal food. On the second day the mice received twenty-six reward pellets plus 5% of their body weight in normal food.

Mice then underwent four days of hole-board training with reward pellets placed in location 3. As in the detailed in phase 2 above, hole-board training consisted of six trials of three minutes each per day, and each day, two hours after the final trial, the mice received 10% of their body weight in normal food. On the fifth day, the mice received a single extra training session with bait placed in location 3 before undergoing an unbaited probe trial (probe trial #3.1) that afternoon. Mice then received their daily light stimulation before receiving twenty reward pellets plus 10% of their body weight in normal food.

### Phase 5 - Reversal training with constant light (location 4)

Mice were then allowed to rest for two days as in phase 4, during which time the light stimulation was received daily. Following the rest period, mice underwent hole-board training for four days following the same protocol as used in phase 4, except that new holes were baited (location 4). As detailed for phase 4, the mice underwent a single additional training for location 4 on the fifth day before undergoing an unbaited probe trial (probe trial #4.1). The probe trial was followed by the two hours of constant light stimulation, and mice given twenty reward pellets plus 10% of their body weight in normal food.

### Experiment - hole-board training with flickering light

### Flickering light exposure

After the last probe trial, the mice were separated into two groups. Group 1 received flickering light exposure at 60 Hz daily, whereas group 2 received constant light (700 lux) daily. During phase 6, mice received five minutes of light exposure on rest days, or for the duration of the training trials (no light exposure was received during the probe trials).

### Phase 6 - Hole-board training with flickeringlconstant light (location 5)

Mice were allowed to rest for two days after the last probe trial. On the first rest day, mice received ten reward pellets plus 15% of their bodyweight in normal food. On the second rest day the mice received twenty-six reward pellets plus 5% of their bodyweight in normal food.

Hole-board training and the unbaited probe trial (probe trial #5.1) for the new pellet location (location 5) were then carried out as detailed in phase 4 except that; 1) the mice received light exposure *during* the training trials, and 2) that half the mice received flickering light exposure, rather than constant light exposure.

After probe trial #5.1, mice were allowed to rest for two days as detailed above. After resting, the mice were subjected to a final unbaited probe trial (probe trial #5.2) with no light stimulation, to determine whether the mice retained the memory of the bait locations.

### Statistical Analysis

Data was analysed using a video tracking system for detecting nose pokes per hole zone. One mouse from Group 1 was removed from analysis due to abnormal movement behaviour (the mouse remained mostly stationary). Working memory index was calculated by dividing (= 1st bout into baited hole/(1st bout + re-bout)).

### Results

The 60 Hz light-exposed animals showed a significant improvement in their working memory, evidenced by the increased number of bouts into baited holes compared to control mice (Figure 11A). Animals exposed to 60 Hz light also performed more total bouts into holes than control mice (Figure 11B). Furthermore, mice exposed to 60 Hz light also had a significant improvement in memory preservation and retrieval as evidenced by the improved performance in probe trial 2 compared to probe trial 1, whereas no memory preservation or retrieval was visible in control mice (Figure 11C).

### Example 3 - Effect of flickering light stimulation on depression behaviours

In a further example, mice are exposed to unpredictable chronic mild stress (UCMS), using protocols such as those described by Burstein and Doron (The Unpredictable Chronic Mild Stress Protocol for Inducing Anhedonia in Mice. J. Vis. Exp. (140), e58184, doi:10.3791/58184 (2018)) and Frisbee et al. (An Unpredictable Chronic Mild Stress Protocol for Instigating Depressive Symptoms, Behavioral Changes and Negative Health Outcomes in Rodents. J. Vis. Exp. (106), e53109, doi:10.3791/53109 (2015)). Mice exposed to UCMS typically display depression-like behavioural phenotypes such as changes in physical activity, increased anxiety, decreased responsiveness to rewards (anhedonia), and increased corticosteroid levels. As a result, UCMS mice show increased anxiety-related behaviours in the elevated plus maze, have a significant increase in their levels of corticosteroids, and require more time (and produce more errors) in the reversal-learning phase of Intellicage experiments.

In this example, UCMS mice (i.e. mice exposed to a stress protocol as described above) are subjected to flickering light treatment at 60 Hz, as described above (see e.g. Example 1). These mice are then tested in a learning and reversal-learning mouse model, i.e. the Intellicage system as described in Example 1.

Exposure of chronically stressed mice to 60 Hz light stimulation is expected to improve performance in Intellicage experiments compared to non-light stimulated chronically stressed controls. Chronically stressed mice exposed to 60 Hz light stimulation may show similar or improved performance in Intellicage experiments compared to non-stressed controls. The improved performance in the Intellicage experiments may correspond to an improvement in working memory, learning, reversal learning, and spatial memory in chronically stressed mice.

In further studies, chronically stressed mice are subjected to flickering light treatment at 60 Hz and then tested in elevated plus maze experiments. Exposure of the chronically stressed mice to 60Hz light stimulation is expected to reduce the anxiety behaviours of chronically stressed mice during elevated plus maze experiments and to reduce levels of corticosteroids in the chronically stressed mice.

The mechanism for this improvement relates to chronic stress causing depression or anxiety in the mice. The chronic stress, anxiety or depression is associated with a reduction in gamma oscillations. Using light stimulation between 50-70 Hz re-establishes gamma activity in the CNS of chronically stressed mice.

The present application claims priority from European patent application no.s 19204173.9, filed 18 October 2019, and 20194579.7, filed 4 September 2020, the contents of which are incorporated herein by reference. All publications mentioned in the above specification are herein incorporated by reference. Various modifications and variations of the described methods and system of the present invention will be apparent to those skilled in the art without departing from the scope and spirit of the present invention. Although the present invention has been described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims.

Further aspects of the present invention are provided in the following numbered paragraphs:
1. A method for promoting neuronal plasticity in a subject, comprising inducing synchronized gamma oscillations in at least one brain region of the subject; wherein the synchronized gamma oscillations have a frequency of about 50 to about 70 Hz.
2. A method according to paragraph 1, wherein the synchronized gamma oscillations induce removal of the perineuronal net.
3. A method according to paragraph 1 or paragraph 2, comprising inducing the synchronized gamma oscillations by applying a visual stimulus to the subject.
4. A method according to paragraph 3, wherein the visual stimulus comprises a flashing light at about 50 to about 70 Hz, about 55 to about 65 Hz or about 57 to about 63 Hz.
5. A method according to any preceding paragraph, comprising inducing the synchronized gamma oscillations by administering a pharmaceutical composition comprising ketamine to the subject.
6. A method according to any preceding paragraph, wherein the method is used to prevent or treat schizophrenia, bipolar disorder, post-traumatic stress disorder and/or depression.
7. A stimulus-emitting device configured to promote neuronal plasticity by induction of *in vivo* synchronized gamma oscillations in at least one brain region of a subject; wherein the device comprises a light source configured to emit flashing light at a frequency of about 50 to about 70 Hz; and wherein the device is configured to induce synchronized gamma oscillations of a frequency of about 50 to about 70 Hz in the brain region of the subject by means of the flashing light.
8. A stimulus-emitting device according to paragraph 7, wherein the light source is configured to emit flashing light at a frequency of about 55 to about 65 Hz or about 57 to about 63 Hz.
9. A stimulus-emitting device according to paragraph 7 or paragraph 8, wherein the light source comprises an array of light emitting diodes.
10. A stimulus-emitting device according to any of paragraphs 7 to 9, further comprising a timer connected to the light source to enable the light source to emit light for a selected period of time.
11. A stimulus-emitting device according to any of paragraphs 7 to 10, wherein the device is configured to emit light having an intensity of about 1 - 8 × 10¹⁸ photons/cm²/s.
12. A stimulus-emitting device according to any of paragraphs 7 to 11, further comprising a sound source, wherein the sound source is configured to promote the induction of the synchronized gamma oscillations at a frequency of about 50 to about 70 Hz in the subject's brain.
13. A stimulus-emitting device according to paragraph 12, wherein the sound source is configured to emit sound pulses at a frequency of about 50 to about 70 Hz.
14. A stimulus-emitting device according to any of paragraphs 7 to 13, for use in preventing or treating schizophrenia, bipolar disorder and/or depression.
15. Ketamine and/or light pulses at a frequency of about 50 to 70 Hz, for use in (i) inducing synchronized gamma oscillations in at least one brain region of a subject; wherein the synchronized gamma oscillations have a frequency of about 50 to about 70 Hz; (ii) promoting removal of the perineuronal net in a brain region of a subject; and/or (iii) promoting neuronal plasticity in a brain region of a subject.

The invention may also be described by reference to the following numbered paragraphs:-
1. A method for promoting cognitive function in a subject, comprising inducing synchronized gamma oscillations in at least one brain region of the subject by applying a visual stimulus comprising a flashing light at about 50 to about 70 Hz to the subject.
2. A method according to paragraph 1, wherein the synchronized gamma oscillations induce neuronal plasticity and/or removal of the perineuronal net in the subject.
3. A method according to paragraph 1 or paragraph 2, wherein the visual stimulus comprises a flashing light at about 55 to about 65 Hz or about 57 to about 63 Hz.
4. A method according to any preceding paragraph, wherein the cognitive function comprises learning, attention or memory.
5. A method according to any preceding paragraph, wherein the subject is a normal subject and/or the method is non-therapeutic.
6. A method according to any preceding paragraph, wherein the subject is experiencing stress, preferably chronic stress.
7. A stimulus-emitting device configured to promote neuronal plasticity by induction of in vivo synchronized gamma oscillations and removal of the perineuronal net in at least one brain region of a subject; wherein the device comprises a light source configured to emit flashing light at a frequency of about 50 to about 70 Hz; and wherein the device is configured to induce synchronized gamma oscillations of a frequency of about 50 to about 70 Hz in the brain region of the subject by means of the flashing light.
8. A stimulus-emitting device according to paragraph 7, wherein the light source is configured to emit flashing light at a frequency of about 55 to about 65 Hz or about 57 to about 63 Hz.
9. A stimulus-emitting device according to paragraph 7 or paragraph 8, wherein the light source comprises an array of light emitting diodes.
10. A stimulus-emitting device according to any of paragraphs 7 to 9, further comprising a timer connected to the light source to enable the light source to emit light for a selected period of time; preferably wherein the period of time is less than one hour, 1 to 30 minutes or about 5 minutes.
11. A stimulus-emitting device according to any of paragraphs 7 to 10, wherein the device is configured to emit light having an intensity of about 1 - 8 × 10¹⁸ photons/ cm²/s.
12. A stimulus-emitting device according to any of paragraphs 7 to 11, further comprising a sound source, wherein the sound source is configured to promote the induction of the synchronized gamma oscillations at a frequency of about 50 to about 70 Hz in the subject's brain.
13. A stimulus-emitting device according to paragraph 12, wherein the sound source is configured to emit sound pulses at a frequency of about 50 to about 70 Hz.
14. A stimulus-emitting device according to any of paragraphs 7 to 13, for use in preventing or treating schizophrenia, bipolar disorder and/or depression.
15. A method of operating a stimulus-emitting device as defined in any of paragraphs 7 to 14, comprising (i) generating flashing light at a frequency of about 50 to about 70 Hz and (ii) directing the flashing light towards a subject.
16. A method according to paragraph 15, wherein the subject is a normal subject.
17. A method according to paragraph 15, wherein the subject is suffering from schizophrenia, bipolar disorder and/or depression.
18. A pharmaceutical composition comprising an active agent for use in a method of treating or preventing schizophrenia, anxiety, bipolar disorder and/or depression in a subject; wherein the method further comprises inducing synchronized gamma oscillations in at least one brain region of the subject by applying a visual stimulus comprising a flashing light at about 50 to about 70 Hz to the subject.
19. A pharmaceutical composition for use according to paragraph 18, wherein the active agent comprises an antidepressant, an anxiolytic, a psychedelic, an antipsychotic or a neuroleptic drug.
20. A pharmaceutical composition for use according to paragraph 19, wherein the active agent is selected from the group consisting of selective serotonin reuptake inhibitors (SSRIs), serotonin-norepinephrine reuptake inhibitors (SNRIs), serotonin antagonist and reuptake inhibitors (SARIs), serotonin modulator and stimulators (SMSs), norepinephrine reuptake inhibitors (NRIs), norepinephrine-dopamine reuptake inhibitors (NDRIs), tricyclic antidepressants (TCAs), tetracyclic antidepressants (TeCAs), monoamine oxidase inhibitors (MAOIs), barbiturates, benzodiazepines, carbamates, antihistamines, opioids, psychedelics, typical antipsychotics and atypical antipsychotics.
21. A pharmaceutical composition for use according to paragraph 20, wherein the active agent is selected from the group consisting of fluoxetine, sertraline, citalopram, escitalopram, fluvoxamine, paroxetine; venlafaxine, desvenlafaxine, duloxetine, milnacipran, levomilnacipran; trazodone, nefazodone; vortioxetine, vilazodone; reboxetine, atomoxetine, teniloxazine, viloxazine; bupropion; amitriptyline, amoxapine, desipramine, doxepine ,imipramine, nortriptyline, protriptyline, trimipramine, clomipramine, maprotiline; mirtazapine, amoxapine, maprotiline, mianserin, setiptiline; isocarboxazid, phenelzine, selegiline, tranylcypromine, rasagiline; agomelatine, ketamine, esketamine, lithium, buspirone, modafmil, lamotrigine; haloperidol, loxapine, thioridazone, molindone, thiothixene, fluphenazine, mesoridazine, trifluoperazine, perphenazine, chlorprothixene, pimozide, prochlorperazine, acetophenazine, triflupromazine; aripiprazole, brexpiprazole, olanzapine, quetiapine, risperidone, lurasidone, amisulpride, clozapine, ziprasidone and cariprazine, phenobarbital; alprazolam, bromazepam, chlordiazepoxide, clonazepam, clorazepate, diazepam, flurazepam, lorazepam, oxazepam, temazepam, triazolam, bromdihydrochlorphenylbenzodiazepine; meprobamate, carisoprodol, tybamate, lorbamate; hydroxyzine, chlorpheniramine and diphenhydramine; hydrocodone, fentanyl, buprenorphine; lysergic acid diethylamide (LSD), psilocybin, cannabis, ayahuasca, ololiuqui, 3,4-methylenedioxymethamphetamine (MDMA), mescaline, ibogaine, salvinorin A, 2,5-dimethoxy-4-methylamphetamine (DOM), 2,5-dimethoxy-4-bromophenethylamine (2C-B), 251-NBOMe (2-(4-iodo-2,5-dimethoxyphenyl)-N-[(2-methoxyphenyl)methyl]-ethanamine), and extracts/derivatives and pharmaceutically acceptable salts thereof.
22. A pharmaceutical composition for use according to paragraph 21, wherein the active agent comprises ketamine, esketamine or a psychedelic drug.
23. A pharmaceutical composition for use according to any of paragraphs 18 to 22, wherein the visual stimulus is applied to the subject at intervals between treatments with the active agent, thereby prolonging or sustaining a response to the active agent.
24. A method for treating or preventing schizophrenia, anxiety, bipolar disorder and/or depression in a subject; the method comprising inducing synchronized gamma oscillations in at least one brain region of the subject by applying a visual stimulus comprising a flashing light at about 50 to about 70 Hz to the subject.
25. A method for sustaining or prolonging a response to an active agent used to treat or prevent schizophrenia, anxiety, bipolar disorder and/or depression in a subject; the method comprising inducing synchronized gamma oscillations in at least one brain region of the subject by applying a visual stimulus comprising a flashing light at about 50 to about 70 Hz to the subj ect.
26. A method according to paragraph 24 or paragraph 25, wherein the visual stimulus is applied to the subject for less than one hour daily, preferably for 1 to 30 minutes or about 5 minutes daily.
27. A method according to any of paragraphs 24 to 26, wherein the visual stimulus is applied to the subject during mental or cognitive challenges or exercises, monocular deprivation, fear extinction training, psychosocial therapy, learning and relearning, psychotherapy, behavioural therapy, trauma therapy, or exposure and response prevention (ERP) therapy.
28. A method according to any of paragraphs 25 to 27, wherein the visual stimulus is applied to the subject, preferably daily, between treatments with the active agent.
29. A method according to paragraph 28, wherein the active agent is administered to the subject in a clinically-supervised environment, and/or wherein the visual stimulus is applied to the subject in an unsupervised or home environment.
30. A method according to any of paragraphs 25 to 29, wherein the active agent comprises ketamine, esketamine or a psychedelic drug.
31. A system for promoting neuronal plasticity in at least one brain region of a subject, comprising (i) a stimulus-emitting device according to any of paragraphs 7 to 14, and (ii) a monitoring device for monitoring synchronized gamma oscillations in the brain region of the subj ect.
32. A system according to paragraph 31, further comprising a processor configured to modulate a duration, frequency and/or intensity of the flashing light emitted by the light source in response to detection of synchronized gamma oscillations of a frequency of about 50 to about 70 Hz by the monitoring device.
33. A system according to paragraph 31 or paragraph 32, further comprising a user interface for displaying brain activity detected by the monitoring device to a user.
34. A system according to any of paragraphs 31 to 33, wherein the monitoring device is an el ectroencephal ogram (EEG) apparatus.

## Claims

1. A stimulus-emitting device configured to promote neuronal plasticity by induction of in vivo synchronized gamma oscillations and removal of the perineuronal net in at least one brain region of a subject; wherein the device comprises a light source configured to emit flashing light at a frequency of about 50 to about 70 Hz; and wherein the device is configured to induce synchronized gamma oscillations of a frequency of about 50 to about 70 Hz in the brain region of the subject by means of the flashing light.

2. A stimulus-emitting device according to claim 1, wherein the light source is configured to emit flashing light at a frequency of about 55 to about 65 Hz or about 57 to about 63 Hz.

3. A stimulus-emitting device according to claim 1 or claim 2, wherein the light source comprises an array of light emitting diodes, and/or
wherein the stimulus emitting device further comprises a timer connected to the light source to enable the light source to emit light for a selected period of time, preferably wherein the period of time is less than one hour, 1 to 30 minutes or about 5 minutes and/or
wherein the device is configured to emit light having an intensity of about 1 - 8 × 10¹⁸ photons/ cm²/s.

4. A stimulus-emitting device according to any preceding claim, wherein the stimulus emitting device further comprises a sound source configured to promote the induction of the synchronized gamma oscillations at a frequency of about 50 to about 70 Hz in the subject's brain, preferably
wherein the sound source is configured to emit sound pulses at a frequency of about 50 to about 70 Hz.

5. A stimulus-emitting device configured to promote neuronal plasticity by induction of in vivo synchronized gamma oscillations and removal of the perineuronal net in at least one brain region of a subject; wherein the device comprises a sound source configured to emit an auditory pulse at a frequency of about 50 to about 70 Hz; and wherein the device is configured to induce synchronized gamma oscillations of a frequency of about 50 to about 70 Hz in the brain region of the subject by means of the sound.

6. A system for promoting neuronal plasticity in at least one brain region of a subject, comprising (i) a stimulus-emitting device configured to promote neuronal plasticity by induction of *in vivo* synchronized gamma oscillations and removal of the perineuronal net in at least one brain region of a subject; wherein the device comprises a light source configured to emit flashing light at a frequency of about 50 to about 70 Hz; and wherein the device is configured to induce synchronized gamma oscillations of a frequency of about 50 to about 70 Hz in the brain region of the subject by means of the flashing light, and (ii) a monitoring device for monitoring synchronized gamma oscillations in the brain region of the subject.

7. The system according to claim 6, further comprising (iii) a processor configured to modulate a duration, frequency and/or intensity of the flashing light emitted by the light source in response to detection of synchronized gamma oscillations of a frequency of about 50 to about 70 Hz by the monitoring device, and/or
further comprising a user interface for displaying brain activity detected by the monitoring device to a user, and/or
wherein the monitoring device is an electroencephalogram (EEG) apparatus, and/or
wherein the device further comprises a sound source, wherein the sound source is configured to promote the induction of synchronized gamma oscillations at a frequency of about 50 to about 70 Hz in the subject's brain.

8. A system for promoting neuronal plasticity in at least one brain region of a subject, comprising (i) a stimulus-emitting device configured to promote neuronal plasticity by induction of *in vivo* synchronized gamma oscillations and removal of the perineuronal net in at least one brain region of a subject; wherein the device comprises a sound source configured to emit sound pulses at a frequency of about 50 to about 70 Hz; and wherein the device is configured to induce synchronized gamma oscillations of a frequency of about 50 to about 70 Hz in the brain region of the subject by means of the sound pulses; and (ii) a monitoring device for monitoring synchronized gamma oscillations in the brain region of the subject.

9. The system according to claim 8, further comprising (iii) a processor configured to modulate a duration, frequency and/or intensity of the sound pulses emitted by the sound source in response to detection of synchronized gamma oscillations of a frequency of about 50 to about 70 Hz by the monitoring device, and/or
further comprising a light source, wherein the light source is configured to promote the induction of synchronized gamma oscillations at a frequency of about 50 to about 70 Hz in the subject's brain.

10. A method for promoting cognitive function in a subject, comprising inducing synchronized gamma oscillations in at least one brain region of the subject by applying a visual stimulus comprising a flashing light at about 50 to about 70 Hz to the subject, or a sound stimulus comprising auditory pulses at about 50 to about 70 Hz, wherein the subject is a normal subject and the method is non-therapeutic.

11. A method for promoting neuronal plasticity in a subject, the method comprising inducing synchronized gamma oscillations in at least one brain region of the subject by applying a visual stimulus comprising a flashing light at about 50 to about 70 Hz to the subject, or a sound stimulus comprising auditory pulses at about 50 to about 70 Hz, wherein the subject is a normal subject and the method is non-therapeutic.

12. A method according to claim 10 or 11, wherein the visual stimulus comprises a flashing light at about 55 to about 65 Hz or about 57 to about 63 Hz, and/or wherein the sound stimulus comprises auditory pulses at about 55 to about 65 Hz or about 57 to about 63 Hz.

13. A method according to claim 10, wherein the cognitive function comprises learning, attention or memory, and/or
wherein the subject is experiencing stress.

14. A pharmaceutical composition comprising an active agent for use in a method of treating or preventing a neuropsychiatric disorder in a subject; wherein the method further comprises inducing synchronized gamma oscillations in at least one brain region of the subject by applying a visual stimulus comprising a flashing light at about 50 to about 70 Hz to the subject or a sound stimulus comprising an auditory pulse at about 50 to about 70 Hz to the subject.

15. A pharmaceutical composition for use according to claim 14, wherein the visual or sound stimulus is applied to the subject at intervals between treatments with the active agent, thereby prolonging or sustaining a response to the active agent, and/or
wherein the neuropsychiatric disorder is selected from schizophrenia, anxiety, bipolar disorder and/or depression, and/or
wherein the active agent comprises an antidepressant, an anxiolytic, a psychedelic, an antipsychotic or a neuroleptic drug, preferably
wherein the active agent is selected from the group consisting of selective serotonin reuptake inhibitors (SSRIs), serotonin-norepinephrine reuptake inhibitors (SNRIs), serotonin antagonist and reuptake inhibitors (SARIs), serotonin modulator and stimulators (SMSs), norepinephrine reuptake inhibitors (NRIs), norepinephrine-dopamine reuptake inhibitors (NDRIs), tricyclic antidepressants (TCAs), tetracyclic antidepressants (TeCAs), monoamine oxidase inhibitors (MAOIs), barbiturates, benzodiazepines, carbamates, antihistamines, opioids, psychedelics, typical antipsychotics and atypical antipsychotics, more preferably
wherein the active agent is selected from the group consisting of fluoxetine, sertraline, citalopram, escitalopram, fluvoxamine, paroxetine; venlafaxine, desvenlafaxine, duloxetine, milnacipran, levomilnacipran; trazodone, nefazodone; vortioxetine, vilazodone; reboxetine, atomoxetine, teniloxazine, viloxazine; bupropion; amitriptyline, amoxapine, desipramine, doxepine ,imipramine, nortriptyline, protriptyline, trimipramine, clomipramine, maprotiline; mirtazapine, amoxapine, maprotiline, mianserin, setiptiline; isocarboxazid, phenelzine, selegiline, tranylcypromine, rasagiline; agomelatine, ketamine, esketamine, lithium, buspirone, modafmil, lamotrigine; haloperidol, loxapine, thioridazone, molindone, thiothixene, fluphenazine, mesoridazine, trifluoperazine, perphenazine, chlorprothixene, pimozide, prochlorperazine, acetophenazine, triflupromazine; aripiprazole, brexpiprazole, olanzapine, quetiapine, risperidone, lurasidone, amisulpride, clozapine, ziprasidone and cariprazine, phenobarbital; alprazolam, bromazepam, chlordiazepoxide, clonazepam, clorazepate, diazepam, flurazepam, lorazepam, oxazepam, temazepam, triazolam, bromdihydrochlorphenylbenzodiazepine; meprobamate, carisoprodol, tybamate, lorbamate; hydroxyzine, chlorpheniramine and diphenhydramine; hydrocodone, fentanyl, buprenorphine; lysergic acid diethylamide (LSD), psilocybin, cannabis, ayahuasca, ololiuqui, 3,4-methylenedioxymethamphetamine (MDMA), mescaline, ibogaine, salvinorin A, 2,5-dimethoxy-4-methylamphetamine (DOM), 2,5-dimethoxy-4-bromophenethylamine (2C-B), 251-NBOMe (2-(4-iodo-2,5-dimethoxyphenyl)-N-[(2-methoxyphenyl)methyl]-ethanamine), and extracts/derivatives and pharmaceutically acceptable salts thereof, even more preferably
wherein the active agent comprises ketamine, esketamine or a psychedelic drug.
